# EUROPÄISCHE PATENTANMELDUNG

(11) **EP 1 710 793 A1**
(43) Veröffentlichungstag der Anmeldung: **11.10.2006**
(21) Anmeldenummer: 05102673.0
(22) Anmeldetag: 05.04.2005
(51) Int. Cl.: G11B 7/249, C07D 413/04, C07D 403/04, C07D 277/64, C07D 263/56, C07D 417/04

(54) **Metallchelate und ihre Verwendung in optischen Aufzeichnungsmedien mit hoher Speicherkapazität**

(71) Anmelder: Ciba Specialty Chemicals Holding Inc., 4057 Basel (CH)
(72) Erfinder: Wolleb, Annemarie, 4232 Fehren (CH); Wolleb, Heinz, 4232 Fehren (CH); Bienewald, Frank, 68220 hegenhein (FR); Budry, Jean-Luc, 2842 Rossemaison (CH)

(57) **Zusammenfassung**

Die Erfindung betrifft neue optische Aufzeichnungsmedien, welche bestimmte, neue Metallchelate enthalten und eine ausgezeichnete Aufnahme- und Wiedergabequalität insbesondere bei einer Wellenlänge von 300 bis 500 nm aufweisen.

Verwendet werden Verbindungen der Formel M^{m+}(Xⁿ)ₓ(L₁)(L₂)_{y}(L₃)_{z} (I), worin L₁ und L₂ unabhängig voneinander je für einen Liganden der Formel 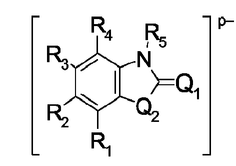 (II) stehen, wobei
L₁ und L₂ über eine Direktbindung oder eine Verbrückung mit C₂-C₁₀Alkylen oder C₂-C₁₀Alkenylen zwischen zwei Radikalen L₁ und L₂ miteinander gebunden sein können;
L₃ unabhängig von L₁ und L₂ für einen weiteren Liganden der Formel (II) steht;
M ein Übergangsmetall der Gruppen 6 bis 12 oder ein Element der Gruppe 13 ist, welches an mindestens einem von R₁, R₂, R₃ und R₄ gebunden ist und zusätzlich mit einem oder mehreren weiteren Liganden koordiniert sein kann und/oder gegebenenfalls zum Ausgleich einer überschüssigen Ladung mit einem oder mehreren weiteren Ionen innerhalb oder ausserhalb der Koordinationssphäre eine elektrostatische Wechselwirkung haben kann;
m eine Zahl 1, 2 oder 3 ist; n eine Ladung -2, -1, +1 oder +2 ist; p eine Zahl 1 oder 2 ist; q, x, y und z unabhängig voneinander eine Zahl 0 oder 1 bedeuten, mit der Bedingung, dass die Summe der Ladungen von M^{m+}, (Xⁿ)ₓ, (L₁), (L₂)_{y} und (L₃)_{z} gleich 0 ist;
Q₁ für CR₆R₇, Q₂ für CR₈R₉, N-OR₈, N-NR₈R₉, O, S oder NR₈, und
Q₃ für CR₁₀R₁₁, C=O, S=O, O=S=O oder P(O)R₁₂ stehen;
und M an mindestens eins von R₁, R₂, R₃ und R₄ komplexiert ist.

Für die genauen Definitionen von R₁ bis R₂₇ sei auf die Beschreibung verwiesen.

Beansprucht sind auch die neuen Verbindungen der Formel (I) sowie deren Verwendung zur technischen Verbesserung von optischen Aufzeichnungsmitteln.

## Beschreibung

Die Erfindung betrifft neue optische Aufzeichnungsmedien, welche bestimmte, neue Metallchelate enthalten und eine ausgezeichnete Aufnahme- und Wiedergabequalität insbesondere bei einer Wellenlänge von 300 bis 500 nm aufweisen. Aufnahme- und Wiedergabe können sehr vorteilhaft bei der gleichen Wellenlänge stattfinden, und die erreichbare Speicherdichte ist deutlich höher als bisher üblich. Die erfindungsgemässen Medien sind zudem vor und nach der Aufzeichnung gut lagerfähig, auch unter harschen Bedingungen wie Exposition zu Sonnen- oder Fluoreszenzröhrenlicht, Hitze und/oder hohe Feuchtigkeit. Sie lassen sich überdies mit üblichen Beschichtungsverfahren, wie Aufschleudern, einfach und gut reproduzierbar herstellen.

JP S62/034957A und JP S63/089388A offenbaren Oxoniumfarbstoffe, welche mit Benzothiazoliden-Gruppen substituiert sind, sowie deren Verwendung in optischen Aufzeichnungsmaterialien. Letztere eignen sich jedoch nur für Laser hoher Wellenlänge.

DE 34 28 459 A offenbart ein optisches Aufzeichnungsmedium enthaltend indigoide Farbstoffe, worin Löcher mit einem He-Ne Laser (633 nm) gebrannt und gelesen werden können.

EP0182236A schlägt reversible optische Aufzeichnungssysteme vor, welche orientierte Monoschichten indigoider oder thioindigoider Farbstoffe enthält. Die Aufnahme erfolgt bei 440 oder 480 nm, wobei eine E/Z Isomerisierung stattfindet und die Schicht farblos wird. Dieses Prinzip ist jedoch mit moderner Technologie inkompatibel, welche auf Änderung der Reflexion basiert.

JP S61/143185 A und JP S61/180238 A seien stellvertretend für eine ganze Reihe von Publikationen erwähnt, welche solche indigoide Systeme durch Komplexierung der Z-Form mit Metallen zu verbessern versucht. Jedoch bleibt das grundsätzliche Problem der unerwünschten Reversibilität unter Einfluss von Licht bestehen. Zudem sind die Schreib- und Lesewellenlängen unterschiedlich.

JP2003/039830A offenbart die Verwendung von Farbmitteln, deren langwelligstes Absorptionsmaximum bei einer Wellenlänge von 340 bis 400nm liegt, in optischen Aufzeichnungsmaterialien, welche bei einer Wellenlänge von maximal 440 nm aufgezeichnet werden. Verbindung HE-3 ist ein Naphthoxazol-Derivat.

WO 02/080164 offenbart optische Aufzeichnungsmaterialien enthaltend Verbindungen mit einem Verhältnis Pyrolysetemperatur zu Absorptionskoeffizient (k) zwischen 950 und 4100°C, unter anderem

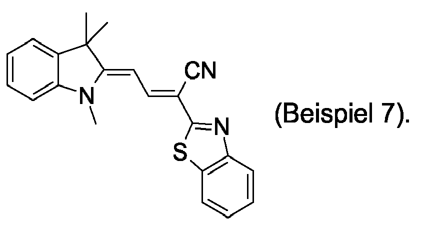

JP 2001/039034A offenbart optische Aufzeichnungsmaterialien enthaltend 2-Aryl-substituierte Benzimidazol-, Benzoxazol- oder Benzothiazolverbindungen. Die optischen Eigenschaften, insbesondere die für höchstmögliche Speicherdichte erforderlichen spektralen Eigenschaften im oder nahe dem UV-Bereich, vermögen jedoch hohen Anforderungen nicht in befriedigendem Mass zu genügen. Die Aufnahme erfolgt mit niedriger Sensitivität bei 430 nm (wünschenswert ist 405 nm) und einer tiefen numerischen Apertur. Zudem genügt die Lichtstabilität hohen Anforderungen in nicht ganz befriedigendem Mass.

US 2004/0 029 040 offenbart optische Aufzeichnungsmedien, welche bei einer Wellenlänge um 405 nm verwendet werden können. Als Aufzeichnungsschicht werden Verbindungen der allgemeinen Formel 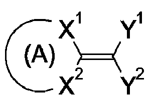 verwendet, wie zum Beispiel 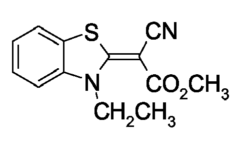 (S-7), (S-8) bis (S-10), (S-23) bis (S-25) und (S-27). Diese Verbindungen können gegebenenfalls durch dissozierbare Gruppen substituiert sein, welche gegebenenfalls als Gegenion Alkali- oder Metallkomplexionen aufweisen, wie Bisbenzol-1,2-dithiolatonickel(III). Diese Medien werden bei einer Geschwindigkeit von nur 3,5 m · s⁻¹ beschrieben.

Obwohl diese Verbindungen angeblich auch in reiner Form verwendet werden können, soll jedoch üblicherweise zur Verbesserung der Lichtstabilität ein ¹O₂- Löscher in Mengen von besonders bevorzugt 5 - 25 Gew.-% zugesetzt werden (Absätze [0065] und [0070]). Dies führt allerdings bei Verwendung eines Lasers der Wellenlänge von 300 bis 500 nm zu einer Verschlechterung der Aufzeichnungsqualität. Wegen der zu hohen Kristallisierungstendenz kann zudem zum Beispiel S-7 nicht zu einer brauchbaren Aufnahmeschicht schleuderbeschichtet werden.

WO 03 / 063151 offenbart optische Aufzeichnungsmedien hoher Speicherdichte enthaltend heterozyklische Azoverbindungen, beispielweise 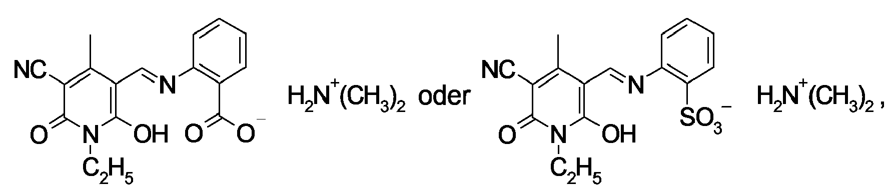 deren Absorptionsmaxima jedoch langwelliger als erwünscht sind, oder auch O,N-koordinierte Metallchelate.

Die bisher bekannten optische Aufzeichnungsmaterialien genügen hohen Anforderungen jedoch nur teilweise, beziehungsweise nicht allen Anforderungen gleichzeitig in voll befriedigendem Mass.

EP 04101 933.2 ist eine Patentanmeldung gemäss Art. 54(3) EPC und Regel 64.3 PCT, welche Metallchelate für optische Aufzeichnungsmedien betrifft.

Ziel der Erfindung ist ein optisches Aufzeichnungsmedium mit hoher Informationsdichte und hoher Datensicherheit. Dieses Aufzeichnungsmedium sollte robust, beständig und einfach zu gebrauchen sein. Darüber hinaus sollte es als Massenprodukt billig herstellbar sein, möglichst kleine und billige Geräte erfordern sowie eine möglichst schnelle, präzise Aufzeichnung der Daten in dauerhaft zuverlässig lesbarer Qualität ermöglichen.

Die Erfindung betrifft daher ein optisches Aufzeichnungsmedium, enthaltend ein Substrat, eine Aufzeichnungsschicht und gegebenenfalls eine reflektierende Schicht, dadurch gekennzeichnet, dass die Aufzeichnungsschicht eine Verbindung der Formel M^{m+}(Xⁿ)ₓ(L₁)(L₂)_{y}(L₃)_{z} (I) enthält, worin L₁ und L₂ unabhängig voneinander je für einen Liganden der Formel 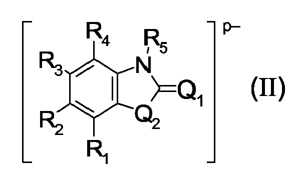 stehen, wobei
L₁ und L₂ über eine Direktbindung oder eine Verbrückung mit C₂-C₁₀Alkylen oder C₂-C₁₀Alkenylen zwischen zwei Radikalen L₁ und L₂ miteinander gebunden sein können;
L₃ unabhängig von L₁ und L₂ für einen weiteren Liganden der Formel (II) steht;
M ein Übergangsmetall der Gruppen 6 bis 12 oder ein Element der Gruppe 13 ist, welches an mindestens einem von R₁, R₂, R₃ und R₄ gebunden ist und zusätzlich mit einem oder mehreren weiteren Liganden koordiniert sein kann und/oder gegebenenfalls zum Ausgleich einer überschüssigen Ladung mit einem oder mehreren weiteren Ionen innerhalb oder ausserhalb der Koordinationssphäre eine elektrostatische Wechselwirkung haben kann;
m eine Zahl 1, 2 oder 3 ist; n eine Ladung -2, -1, +1 oder +2 ist; p eine Zahl 1 oder 2 ist; q, x, y und z unabhängig voneinander eine Zahl 0 oder 1 bedeuten, mit der Bedingung, dass die Summe der Ladungen von M^{m+}, (Xⁿ)ₓ, (L₁), (L₂)_{y} und (L₃)_{z} gleich 0 ist;
Q₁ für CR₆R₇, Q₂ für CR₈R₉, N-OR₈, N-NR₈R₉, O, S oder NR₈, und Q₃ für CR₁₀R₁₁, C=O, S=O, O=S=O oder P(O)R₁₂ stehen;
entweder eins von R₁, R₂, R₃ und R₄, oder zwei von R₁, R₂, R₃ und R₄ in einem Paar R₁ und R₂, R₂ und R₃ oder R₃ und R₄ je unabhängig voneinander (Q₃)_{q}R₁₃ sind, und die weiteren zwei oder drei von R₁, R₂, R₃ und R₄ unabhängig voneinander R₁₄, NR₁₀NR₈R₉, NO₂, SiR₈R₁₅R₁₆, C(R₁₀)=NR₈, C(R₁₀)=N-OR₈, CON(R₁₀)OR₈, CON(R₁₀)NR₈R₉, S(O)R₁₅, S(O)₂-R₁₅, S(O)-OR₈, S(O)N(R₁₀)NR₈R₉, SO₂NR₈R₉, SO₂N(R₁₀)NR₈R₉, SO₃R₈, P(O)R₁₅R₁₆, P(O)R₁₅OR₈, P(O)OR₈OR₉ oder P(O)(NR₈R₉)₂ bedeuten, wobei eins der weiteren zwei oder drei R₁, R₂, R₃ und R₄ darüber hinaus unsubstituiertes oder mit einem oder mehreren, gegebenenfalls identischen oder unterschiedlichen Resten Halogen, OH, OR₁₅, SR₁₅, NH₂, NHR₁₅, NR₁₅R₁₆, Nitro, CN, OCN, SCN, CHO, COR₁₅, CR₁₇OR₁₅OR₁₆, COOH, COOR₁₅, CONH₂, CONHR₁₅, CONR₁₅R₁₆, SO₂R₁₅, P(O)R₁₅R₁₆, P(O)R₁₅OR₁₆ und/oder P(O)OR₁₅OR₁₆ substituiertes C₆-C₁₀Aryl, C₁-C₉Heteroaryl, C₇-C₁₂Aralkyl oder C₂-C₁₂Heteroaralkyl sein kann;
oder zwei der weiteren zwei oder drei R₁, R₂, R₃ und R₄ stehen als Paar R₁ und R₂, R₂ und R₃ oder R₃ und R₄ zu zweit zusammen für unsubstituiertes oder mit NR₁₅R₁₆, SR₁₅, Halogen und/oder OR₁₅ ein- oder mehrfach substituiertes C₂-C₁₀Alkylen oder C₂-C₁₀Alkenylen, wobei ein zusätzlicher Ring gebildet wird, welcher bevorzugt nicht durchkonjugiert ist;
R₅, R₈, R₉, R₁₀ und R₁₁ unabhängig voneinander und unabhängig von R₁ bis R₄, R₆, R₇ und R₁₂ bis R₁₉ für Wasserstoff, für unsubstituiertes oder mit NR₁₅R₁₆, SR₁₅, Halogen und/oder OR₁₅ ein- oder mehrfach substituiertes C₁-C₅Alkyl, C₂-C₅Alkenyl, C₂-C₅Alkinyl, C₃-C₅Cycloalkyl, Hetero-C₂-C₅cycloalkyl oder C₃-C₅Cycloalkenyl, oder für unsubstituiertes oder mit einem oder mehreren, gegebenenfalls identischen oder unterschiedlichen Resten Halogen, OH, OR₁₅, SR₁₅, NH₂, NHR₁₅, NR₁₅R₁₆, Nitro, CN, OCN, SCN, CHO, COR₁₅, CR₁₇OR₁₅OR₁₆, COOH, COOR₁₅, CONH₂, CONHR₁₅, CONR₁₅R₁₆, SO₂R₁₅, P(O)R₁₅R₁₆, P(O)R₁₅OR₁₆ und/oder P(O)OR₁₅OR₁₆ substituiertes C₆-C₁₀Aryl, C₁-C₉Heteroaryl, C₇-C₁₂Aralkyl oder C₂-C₁₂Heteroaralkyl stehen; oder R₈ und R₉ und/oder R₁₀ und R₁₁ stehen zusammen für unsubstituiertes oder mit NR₁₅R₁₆, SR₁₅, Halogen und/oder OR₁₅ ein- oder mehrfach substituiertes C₂-C₁₀Alkylen oder C₂-C₁₀Alkenylen, welches durch O oder NR₁₅ unterbrochen sein kann;
R₆ für Wasserstoff, R₁₈, OR₈, SR₈, NR₈R₉; für unsubstituiertes oder mit COR₁₀, COOR₁₀, CONR₈R₉, CN, Halogen und/oder OR₁₅ ein- oder mehrfach substituiertes C₁-C₅Alkyl, C₂-C₅Alkenyl, C₂-C₅Alkinyl, C₃-C₅Cycloalkyl, Hetero-C₂-C₅cycloalkyl oder C₃-C₅Cycloalkenyl steht;
R₇ und R₁₈ unabhängig voneinander und unabhängig von R₁ bis R₆, R₈ bis R₁₇ und R₁₉ für CN, COR₁₀, C(R₁₀)=NR₂₀, COOR₁₀, CONR₈R₉, SOR₁₂, SO₂R₁₂ oder S0₂NR₈R₉ stehen;
wobei R₆ und R₇ gegebenenfalls über eine Direktbindung, O, S, NR₈, C=O, C=S oder C=NR₈ zusammen gebunden sein können, so dass sich für Q₁ ein 5-, 6- oder 7-gliedriger Ring ergibt;
R₁₂ für OR₈, SR₈, NR₈R₉ oder R₁₁ steht;
R₁₃ für O⁻, S⁻ oder N⁻R₂₁ steht;
R₁₄, gegebenenfalls jedes R₁₄ unabhängig von anderen R₁₄, für Wasserstoff, Halogen, OR₁₉, SR₁₉, NR₈R₉, COR₁₀, COOR₁₀, CONR₈R₉, CN oder für unsubstituiertes oder mit NR₁₅R₁₆, SR₁₅, Halogen und/oder OR₁₅ ein- oder mehrfach substituiertes C₁-C₅Alkyl, C₂-C₅Alkenyl, C₂-C₅Alkinyl, C₃-C₅Cycloalkyl, Hetero-C₂-C₅Cycloalkyl oder C₃-C₅Cycloalkenyl steht;
R₁₅, R₁₆ und R₁₇ unabhängig voneinander für unsubstituiertes oder mit NR₂₂R₂₃, SR₂₂, Halogen und/oder OR₂₂ ein- oder mehrfach substituiertes C₁-C₅Alkyl, C₂-C₅Alkenyl, C₂-C₅Alkinyl, C₃-C₅Cycloalkyl, Hetero-C₂-C₅cycloalkyl oder C₃-C₅Cycloalkenyl stehen, oder R₁₅ und R₁₆ stehen zusammen für unsubstituiertes oder mit NR₂₂R₂₃, SR₂₂, Halogen und/oder OR₂₂ ein- oder mehrfach substituiertes C₂-C₁₀Alkylen oder C₂-C₁₀Alkenylen, welches durch O oder NR₂₂ unterbrochen sein kann;
R₁₉ für Wasserstoff, COR₁₀, COOR₂₄, CR₈OR₉OR₁₀, CN, CONR₈R₉, SO₂R₂₄, P(O)R₂₄R₂₅, P(O)R₂₄OR₂₅, P(O)OR₂₄OR₂₅ oder für unsubstituiertes oder mit NR₁₅R₁₆, SR₁₅, Halogen und/oder OR₁₅ ein- oder mehrfach substituiertes C₁-C₅Alkyl, C₂-C₅Alkenyl, C₂-C₅Alkinyl, C₃-C₅Cycloalkyl, Hetero-C₂-C₅cycloalkyl oder C₃-C₅Cycloalkenyl steht;
R₂₀ für NR₈R₉, OR₉, SR₉, R₈ oder R₂₁ steht;
R₂₁ für COR₁₀, COOR₂₄, CN, CONR₈R₉, SO₂R₂₄, P(O)R₂₄R₂₅, P(O)R₂₄OR₂₅ oder P(O)OR₂₄OR₂₅ steht;
R₂₂ und R₂₃ unabhängig voneinander und unabhängig von R₁ bis R₁₉ für Wasserstoff, für unsubstituiertes oder mit Halogen, OR₂₆ und/oder NR₂₆R₂₇ ein- oder mehrfach substituiertes C₁-C₅Alkyl, C₂-C₅Alkenyl , C₂-C₅Alkinyl, C₃-C₅Cycloalkyl, Hetero-C₂-C₅cycloalkyl oder C₃-C₅Cycloalkenyl, oder für unsubstituiertes oder mit einem oder mehreren, gegebenenfalls identischen oder unterschiedlichen Resten Halogen, OR₂₆, NR₂₆R₂₇, Nitro, CN, OCN, SCN, COR₂₆, CR₂₆OR₂₈OR₂₉, COOR₂₈, CONR₂₆R₂₇, SO₂R₂₈, P(O)R₂₈R₂₉, P(O)R₂₈OR₂₉ und/oder P(O)OR₂₈OR₂₉ substituiertes Phenyl, C₁-C₄Heteroaryl, Benzyl oder C₂-C₅Heteroaralkyl stehen; oder R₂₂ und R₂₃ stehen zusammen für unsubstituiertes oder mit NR₂₆R₂₇, SR₂₆, Halogen und/oder OR₂₆ ein- oder mehrfach substituiertes C₂-C₅Alkylen oder C₂-C₅Alkenylen, welches durch O oder NR₂₆ unterbrochen sein kann;
R₂₄ und R₂₅ unabhängig voneinander für unsubstituiertes oder mit NR₂₆R₂₇, SR₂₆, Halogen und/oder OR₂₆ ein- oder mehrfach substituiertes C₁-C₅Alkyl, C₂-C₅Alkenyl, C₂-C₅Alkinyl, C₃-C₅Cycloalkyl, Hetero-C₂-C₅cycloalkyl oder C₃-C₅Cycloalkenyl stehen, oder R₂₄ und R₂₅ stehen zusammen für unsubstituiertes oder mit NR₂₆R₂₇, SR₂₆, Halogen und/oder OR₂₆ ein- oder mehrfach substituiertes C₂-C₁₀Alkylen oder C₂-C₁₀Alkenylen, welches durch O oder NR₂₆ unterbrochen sein kann;
R₂₆ und R₂₇ unabhängig voneinander für Wasserstoff, Methyl oder Ethyl, sowie R₂₈ und R₂₉ unabhängig voneinander für Methyl oder Ethyl stehen; oder R₂₆ und R₂₇ und/oder R₂₈ und R₂₉ stehen zusammen für unsubstituiertes oder durch Methyl und/oder Ethyl 1- bis 5-fach substituiertes 1,5-Pentylen, 3-Oxa-1,5-pentylen oder 3-Oxa-1,5-pentylen; und
Xⁿ ein Gegenion bedeutet.

Halogen ist Chlor, Brom, Fluor oder Jod, bevorzugt Fluor, Chlor oder Brom, besonders bevorzugt Fluor (zum Beispiel in Trifluormethyl, α,α-Difluorethyl, β,β,β-Trifluorethyl oder perfluorierten Alkylgruppen wie Heptafluorpropyl).

Alkyl, Cycloalkyl, Alkenyl oder Cycloalkenyl kann geradkettig oder verzweigt, monozyclisch oder polyzyclisch sein. Alkyl ist beispielsweise Methyl, geradkettiges C₂-C₅Alkyl oder bevorzugt verzweigtes C₃-C₅Alkyl. Alkenyl ist beispielsweise geradkettiges C₂-C₅Alkenyl oder verzweigtes C₃-C₅Alkenyl. C₁-C₅Alkyl ist daher zum Beispiel Methyl, Ethyl, n-Propyl, Isopropyl, n-Butyl, sec.-Butyl, Isobutyl, tert.-Butyl, n-Pentyl, 2-Pentyl, 3-Pentyl oder 2,2-Dimethylpropyl. C₃-C₅Cycloalkyl ist zum Beispiel Cyclopropyl, Cyclobutyl oder Cyclopentyl.

Heterocycloalkyl ist Cycloalkyl, worin eins oder mehrere, jedoch nicht alle C-Atome durch Elemente der Gruppen 13 bis 16 ersetzt sind, beispielsweise durch N-, O- oder S-Atome. Bevorzugt ist Oxa- und Thiacycloalkyl, wie zum Beispiel Epoxide, Episulfide, Oxetyl, Thietyl und Tetrahydrofuryl, oder auch N-alkylierte Aziridine, wie 2-(1-Aza-1-ethyl)-cyclopropyl oder 2-(1 -Aza-1-methyl)-cydopropyl-methyl.

C₂-C₅Alkenyl oder C₃-C₅Cycloalkenyl sind C₂-C₅Alkyl beziehungsweise C₃-C₅Cycloalkyl, welches ein- oder zweifach ungesättigt ist, wobei zwei Doppelbindungen gegebenenfalls isoliert oder konjugiert sein können, zum Beispiel Vinyl, Allyl, 2-Propen-2-yl, 2-Buten-1-yl, 3-Buten-1-yl, 1,3-Butadien-2-yl, 2-Cyclobuten-1-yl, 2-Penten-1-yl, 3-Penten-2-yl, 2-Methyl-1-buten-3-yl, 2-Methyl-3-buten-2-yl, 3-Methyl-2-buten-1-yl, 1,4-Pentadien-3-yl oder2-Cyclopenten-1-yl.

Alkylen kann ebenfalls geradkettig oder verzweigt sein und ist beispielsweise Methylen, geradkettiges C₂-C₁₀Alkylen oder bevorzugt C₃-C₁₀Alkylen, welches gegebenenfalls ein- oder mehrfach verzweigt sein kann. C₁-C₁₀Alkylen ist daher zum Beispiel Methylen, Methyliden, Ethylen, 1,2-Propylen, 1,3-Propylen, 2-Methyl-1,2-propylen, 2-Methyl-1,3-Propylen, 3-Methyl-1,3-Propylen, 1,2-Butylen, 1,3-Butylen, 2,3-Butylen, 1,4-Butylen oder ein beliebiges Isomer von Pentylen, Hexylen, Heptylen, Octylen, Nonylen oder Decylen, wie die verschiedenen Stereoisomere von 2,3,4,5-Tetramethyl-2,5-hexylen.

C₂-C₁₀Alkenylen ist C₂-C₁₀Alkylen, welches ein- oder zweifach ungesättigt ist, wobei zwei Doppelbindungen gegebenenfalls isoliert oder konjugiert sein können, sofern jedoch keine durchgehende Konjugation entsteht. Die Konjugation des π-Systems muss gegebenenfalls durch mindestens einen voll gesättigten C-Atom in der Verbrückung zwischen den beiden freien Valenzen des Alkenylendiradikals unterbrochen sein. Dieser voll gesättigte C-Atom kann dabei selbst eine freie Valenz aufweisen, oder auch anderswo in der Verbrückung zwischen diesen beiden freie Valenzen aufweisenden C-Atomen liegen. C₂-C₆Alkenylen ist daher zum Beispiel 1-Propen-1,3-ylen, 1-Buten-1,3-ylen, 1-Buten-1,4-ylen, 2-Buten-1,4-ylen, 3-Buten-1,3-ylen, 1-Penten-1,3-ylen, 1-Penten-1,4-ylen, 1-Penten-1,5-ylen, 1-Penten-2,3-ylen, 1-Penten-2,4-ylen, 1-Penten-2,5-ylen, 1-Penten-3,4-ylen, 1-Penten-3,5-ylen, 1-Penten-4,5-ylen, 2-Penten-1,3-ylen, 2-Penten-1,4-ylen, 2-Penten-1,5-ylen, 2-Penten-2,4-ylen, 2-Penten-2,5-ylen, 2-Penten-3,4-ylen, 2-Penten-3,5-ylen, 2-Penten-4,5-ylen, 1,3-Pentadien-1,5-ylen, 1,3-Pentadien-2,5-ylen, 1,3-Pentadien-3,5-ylen, 1,3-Pentadien-4,5-ylen, 1,4-Pentadien-1,3-ylen, 1,4-Pentadien-1,4-ylen, 1,4-Pentadien-1,5-ylen, 1,4-Pentadien-2,4-ylen, 1-Hexen-1,3-ylen, 1-Hexen-1,4-ylen, 1-Hexen-1,5-ylen, 1-Hexen-1,6-ylen, 1-Hexen-2,3-ylen, 1-Hexen-2,4-ylen, 1-Hexen-2,5-ylen, 1-Hexen-2,6-ylen, 1-Hexen-3,4-ylen, 1-Hexen-3,5-ylen, 1-Hexen-3,6-ylen, 1-Hexen-4,5-ylen, 1-Hexen-4,6-ylen, 1-Hexen-5,6-ylen, 2-Hexen-1,3-ylen, 2-Hexen-1,4-ylen, 2-Hexen-1,5-ylen, 2-Hexen-1,6-ylen, 2-Hexen-2,4-ylen, 2-Hexen-2,5-ylen, 2-Hexen-2,6-ylen, 2-Hexen-3,4-ylen, 2-Hexen-3,5-ylen, 2-Hexen-3,6-ylen, 2-Hexen-4,5-ylen, 2-Hexen-4,6-ylen, 2-Hexen-5,6-ylen, 3-Hexen-1,2-ylen, 3-Hexen-1,3-ylen, 3-Hexen-1,4-ylen, 3-Hexen-1,5-ylen, 3-Hexen-1,6-ylen, 3-Hexen-2,3-ylen, 3-Hexen-2,4-ylen, 3-Hexen-2,5-ylen, 3-Hexen-2,6-ylen, 3-Hexen-3,5-ylen, 3-Hexen-3,6-ylen, 3-Hexen-4,5-ylen, 3-Hexen-4,6-ylen, 3-Hexen-5,6-ylen, 1,3-Hexadien-1,5-ylen, 1,3-Hexadien-1,6-ylen, 1,3-Hexadien-2,5-ylen, 1,3-Hexadien-2,6-ylen, 1,3-Hexadien-3,5-ylen, 1,3-Hexadien-3,6-ylen, 1,3-Hexadien-4,5-ylen, 1,3-Hexadien-4,6-ylen, 1,3-Hexadien-5,6-ylen, 1,4-Hexadien-1,3-ylen, 1,4-Hexadien-1,4-ylen, 1,4-Hexadien-1,5-ylen, 1,4-Hexadien-1,6-ylen, 1,4-Hexadien-2,3-ylen, 1,4-Hexadien-2,4-ylen, 1,4-Hexadien-2,5-ylen, 1,4-Hexadien-2,6-ylen, 1,4-Hexadien-3,4-ylen, 1,4-Hexadien-3,5-ylen, 1,4-Hexadien-3,6-ylen, 1,4-Hexadien-4,6-ylen, 1,4-Hexadien-5,6-ylen, 1,5-Hexadien-1,3-ylen, 1,5-Hexadien-1,4-ylen, 1,5-Hexadien-1,5-ylen, 1,5-Hexadien-1,6-ylen, 1,5-Hexadien-2,3-ylen, 1,5-Hexadien-2,4-ylen, 1,5-Hexadien-2,5-ylen, 1,5-Hexadien-3,4-ylen, 2,4-Hexadien-1,2-ylen, 2,4-Hexadien-1,3-ylen, 2,4-Hexadien-1,4-ylen, 2,4-Hexadien-1,5-ylen oder 2,4-Hexadien-1,6-ylen, jedoch beispielsweise weder 1,2-Ethylen noch Butadien-1,3-ylen noch Butadien-1,4-ylen. Dasselbe Prinzip gilt analog auch für höhere Analoge davon sowie für C₇-C₁₀Alkenylen.

C₇-C₁₂Aralkyl ist zum Beispiel Benzyl, 2-Benzyl-2-propyl, β-Phenyl-ethyl, α,α-Dimethylbenzyl, ω-Phenyl-butyl oder ω-Phenyl-hexyl, bevorzugt Benzyl. Ist C₇-C₁₂Aralkyl substituiert, so können sowohl der Alkyl- als auch am Aryl-Teil der Aralkyl-Gruppe substituiert sein, wobei letztere Alternative bevorzugt ist.

C₆-C₁₀Aryl ist zum Beispiel Phenyl, Naphthyl oder Biphenylyl, bevorzugt Phenyl.

C₁-C₉Heteroaryl ist ein ungesättigtes oder aromatisches Radikal mit 4n+2 konjugierten π-Elektronen, beispielsweise 2-Thienyl, 2-Furyl, 2-Pyridyl, 2-Thiazolyl, 2-Oxazolyl, 2-Imidazolyl, Isothiazolyl, Thiadiazolyl, Triazolyl, Tetrazolyl oder ein beliebiges sonstiges, aus Thiophen-, Furan-, Pyridin, Thiazol, Thiadiazol, Oxazol, Imidazol, Isothiazol, Triazol, Pyridin- und Benzolringen bestehendes, unsubstituiertes oder mit 1 bis 6 Ethyl, Methyl, Ethylen und/oder Methylen substituiertes Ringsystem, zum Beispiel Benzotriazolyl, bei N-Heterozyklen gegebenenfalls auch in Form derer N-Oxide.

C₂-C₁₂Heteroaralkyl ist beispielsweise mit C₁-C₁₁Heteroaryl substituiertes C₁-C₈Alkyl.

Darüber hinaus können Aryl und Aralkyl auch mit einem Metall verbundene aromatische Gruppen sein, zum Beispiel in Form an sich bekannter Metallocene von Übergangsmetallen, ganz besonders bevorzugt 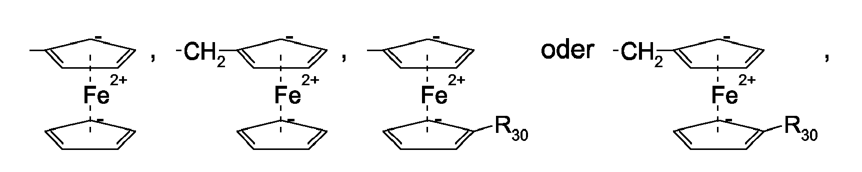 worin R₃₀ für CH₂OH, CH₂OR₁₇ oder COOR₁₇ steht.

Die Stereochemie der Doppelbindung zu CR₆R₇ kann sowohl E als auch Z sein.

Bei der Verbindung der Formel (I) kann es sich auch um ein Dimer oder Oligomer handeln, wobei zwei oder mehr Radikale der Formel (I) durch Direktbindungen zwischen Substituenten oder durch Verbrückungen mit C₂-C₁₀Alkylen oder C₂-C₁₀₋Alkenylen gemäss den zuvor gegebenen Definitionen miteinander gebunden sind. Allerdings soll auch in diesem Fall zweckmässig keine durchgehende Konjugation entstehen. Oligomere bestehen bevorzugt aus 3, 4 oder 5 Radikalen der Formel (I) und können zyklisch sein oder als Endgruppen nicht metallisierte, mit nur einem Radikal der Formel (I) sowie anderen Liganden koordinierte und /oder nicht verbrückte Radikale der Formel (I) aufweisen.

M ist zweckmässig ein Übergangsmetall der Gruppen 6 bis 12, bevorzugt ein Übergangsmetall der Gruppen 8 bis 12, insbesondere ein Übergangsmetall der Gruppen 9 bis 11 (neue IUPAC-Nomenklatur), beispielsweise Au, Cd, Co, Cu, Cr, Ir, Mn, Mo, Ni, Fe, Os, Pd, Pt, Re, Rh, Ru, W oder Zn, besonders bevorzugt Co, Cu oder Ni, insbesondere Co(II), Cu(II) oder Ni(II). In Verbindungen der Formel (I), worin z gleich 1 ist, handelt es sich beim Übergangsmetallkation bevorzugt um ein relativ grosses Kation, wie Ir³⁺ oder Rh³⁺.

Je nach Anzahl Elektronen in der äussersten d-Schale können solche Übergangsmetalle mit weiteren Liganden koordiniert sein. Bei den weiteren Liganden handelt es sich beispielsweise um bekannte Verbindungen, zum Beispiel Ammoniak, Acetylaceton, Wasser, Amine, Polyamine, Alkohole, Polyalkohole oder Olefine.

Die Verbindungen der Formel (I) sind zweckmässig elektronisch neutral, was die Präsenz von Kationen und Anionen keineswegs ausschliesst sofern deren Ladungen sich gegenseitig ausgleichen. Dabei kann es sich gegebenenfalls sowohl um lonenpaare als auch um Zwitterionen handeln.

Beim Gegenion kann es sich um ein Proton, ein Metall-, Ammonium- oder Phosphonium-Kation, um ein anorganisches, organisches oder metallorganisches Anion, oder auch um ein positiv oder negativ geladenes organisches oder metallorganisches Chromophor handeln.

Anione sind beispielsweise Hydroxid, Oxid, Fluorid, Chlorid, Bromid, lodid, Perchlorat, Periodat, Carbonat, Hydrogencarbonat, Sulfat, Hydrogensulfat, Phosphat, Hydrogenphosphat, Dihydrogenphosphat, Hexafluorophosphat, Hexafluoroantimonat, Tetrafluoroborat, Acetat, Oxalat, Malonat, Methansulfonat, Trifluormethansulfonat, Tosylat, Methylsulfat, Phenolat, Benzoat oder ein negativ geladener Metallkomplex, zum Beispiel ein Bisdithiolat eines Übergangsmetalles, wie Co²⁺, Co³⁺, Ni²⁺, Ni³⁺ oder insbesondere Cu²⁺.

Metall-, Ammonium- oder Phosphonium-Katione sind beispielsweise Li⁺, Na⁺, K⁺, Mg²⁺, Ca²⁺, Cu²⁺, Ni²⁺ , Fe²⁺, Co²⁺, Co³⁺, Zn²⁺, Sn²⁺, Methylammonium, Ethylammonium, Pentadecylamonium, Isopropylammonium, Dicyclohexylammonium, Tetramethylammonium, Tetraethylammonium, Tetrabutylammonium, Benzyltrimethylammonium, Benzyltriethylammonium, Methyltrioctylammonium, Tridodecylmethylammonium, 1,2-Ethylendiammonium, 1,2-Propylendiammonium, 1,4-Butylendiammonium, 1,6-Hexylendiammonium, 1,8-Octylendiammonium, Tetrabutylphosphonium, Tetraphenylphosphonium, Butyltriphenylphosphonium oder Ethyltriphenylphosphonium, oder auch protoniertes Primen 81 R™ oder Rosin Amin D™. Bevorzugt sind H, Na⁺, K⁺, NH₄⁺, primäres, sekundäres, tertiäres oder quaternäres Ammonium sowie kationische Chromophore.

Als positiv geladene organische Chromophore können beliebige im Bereich von 300 bis 1500 nm, insbesondere im Bereich von 300 bis 800 nm absorbierende Kationen verwendet werden. Der Fachmann wird bevorzugt insbesondere Chromophorkationen auswählen, welche schon bisher zur Verwendung in optischen Informationsmedien vorgeschlagen wurden, beispielsweise Cyanin-, Xanthen-, Dipyrromethen-, Styryl-, Triphenylmethin-, Azo-, Metallkomplex-, Chinondiimmonium-, Bipyridinium- und andere Kationen. Bevorzugt sind Cyanin-, Xanthen-, Dipyrromethen-, Azometallkomplex- und Styryl-Kationen. Weitere in kationischer Form verwendbare Chromophore sind WO-01/75873 zu entnehmen, wobei diese Beispiele keineswegs als einschränkende Auswahl anzusehen sind.

Ist nur eins von R₁, R₂, R₃ und R₄ gleich (Q₃)_{q}R₁₃, so handelt es sich bevorzugt um R₁ in Kombination mit Q₂ gleich O oder S. In diesem Fall kann das Übergangsmetall M zusätzlich mit Q₂ komplexieren.

Besonders bevorzugt sind jedoch Liganden der Formel (II), worin das Übergangsmetall M mit zwei vicinalen Resten R₁, R₂, R₃ oder R₄ komplexiert. Dabei können entweder beide komplexierende Reste als (Q₃)_{q}R₁₃an R₁₃ eine negative Ladung (O⁻, S⁻ oder N⁻) haben, oder bevorzugt nur eins davon, wobei der Komplex mit einem Heteroatom N, O oder S auf einem vicinalen Rest gebildet wird. Es bildet sich dabei inklusiv der Koordination mit dem Übergangsmetall M ein 5-, 6- oder 7-gliedriger Ring, besonders bevorzugt ein 6-gliedriger Ring. Von den Resten R₁, R₂, R₃ oder R₄, welche nicht (Q₃)_{q}R₁₃ sind, ist bevorzugt ein zu (Q₃)_{q}R₁₃ vicinal stehender Rest R₁, R₂, R₃ oder R₄ gleich CH₂NR₁₅R₁₆.

Dementsprechend kann die Erfindung in verschiedenen Ausführungsvarianten ausgeübt werden, wobei diejenigen mit den folgenden Teilstrukturen M^{m+}(L₁) von besonderem Interesse sind (q kann dabei jeweils 0 oder 1 sein) : 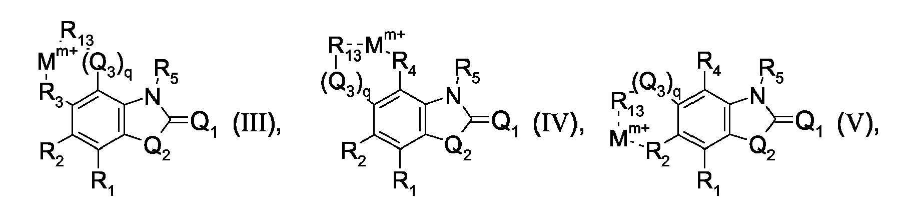 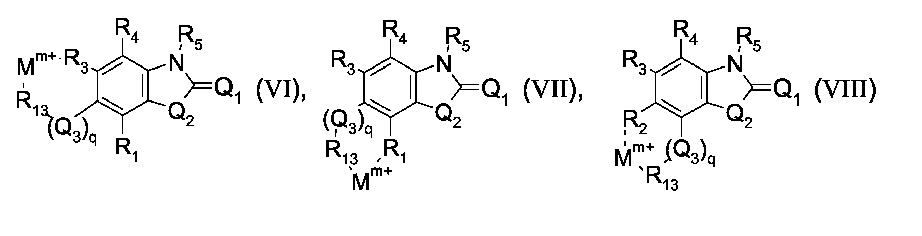 oder

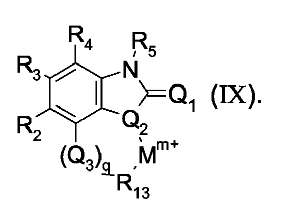

Die Definitionen gemäss den Formeln (I) und (II) gelten selbstverständlich auch für die Formel (III), (IV), (V), (VI), (VII), (VIII) und (IX). Dies ist auch der Fall für die nachfolgend angegebenen Bevorzugungen.
R₅, R₈ und R₉ sind bevorzugt unabhängig voneinander Wasserstoff, Methyl, Ethyl oder Benzyl;
R₆ ist bevorzugt Wasserstoff oder R₁₈;
R₁₀ ist bevorzugt unsubstituiertes oder mit Halogen und/oder OR₁₅ ein- oder mehrfach substituiertes C₁-C₅Alkyl, C₂-C₅Alkenyl, C₂-C₅Alkinyl, C₃-C₅Cycloalkyl, Hetero-C₂-C₅cycloalkyl oder C₃-C₅Cycloalkenyl, besonders bevorzugt Wasserstoff oder C₁-C₃Alkyl, ganz besonders bevorzugt Wasserstoff, Methyl oder Ethyl;
R₁₅ und R₁₆ sind bevorzugt unabhängig voneinander C₁-C₅Alkyl oder C₂-C₅Alkenyl; bevorzugt sind m die Zahl 2, x die Zahl 0, y die Zahl 1, und z die Zahl 0;
L₁ und L₂ sind bevorzugt Liganden der Formel 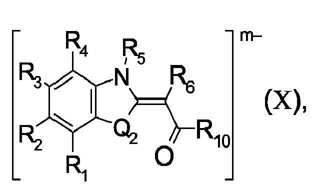 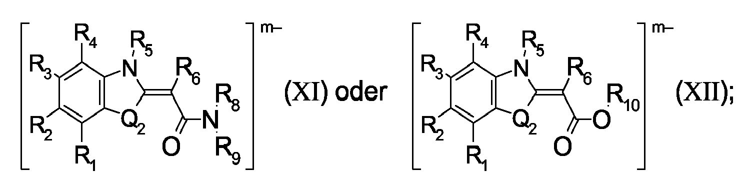 und/oder
Q₂ ist bevorzugt CR₈R₉, S, O oder NR₁₉, besonders bevorzugt S, O oder NR₁₉, ganz besonders bevorzugt S.

Besonders interessant sind diejenige Verbindungen, worin L₁ und L₂ das gleiche Komplexierungsmuster mit dem Metall M^{m+} haben, das heisst worin M^{m+}(L₁) und M^{m+}(L₂) die gleiche Teilstruktur (111), (IV), (V), (VI), (VII), (VIII) oder (IX) aufweisen, wobei L₁ und L₂ identisch oder auch unterschiedlich sein können.

Alkyl, Alkenyl, Alkinyl, Cycloalkyl, Heterocycloalkyl oder Cycloalkenyl an beliebiger Stelle ist bevorzugt Methyl, Ethyl, n-Propyl, Isopropyl, Vinyl, Allyl, Propargyl, Cyclopropyl, 2-Oxacyclopropyl oder 2-Thiacyclopropyl, besonders bevorzugt Trifluormethyl, α,α-Difluorethyl, β,β,β-Trifluorethyl oder Heptafluorpropyl, ganz besonders bevorzugt als R₁₀.

Diese Bevorzugungen gelten sowohl einzeln als auch in beliebiger Kombination, insbesondere auch mit allen Ausführungsvarianten (III) bis (XII). In der Regel weisen alle erfindungsgemässen Verbindungen desto vorteilhaftere Eigenschaften, je mehr bevorzugte Einzelmerkmale sie aufweisen.

Die beachtlich hohe Vielseitigkeit der Erfindung ist anhand folgender rein illustrativer Beispiele erkennbar:

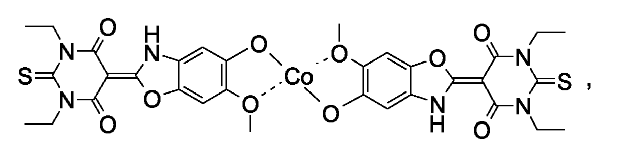

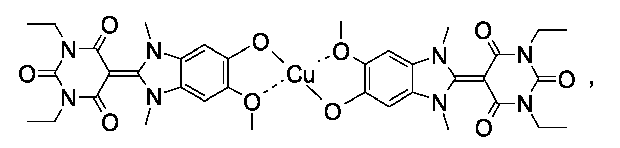

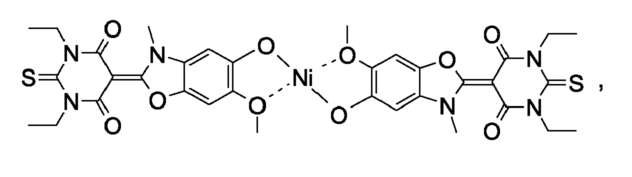

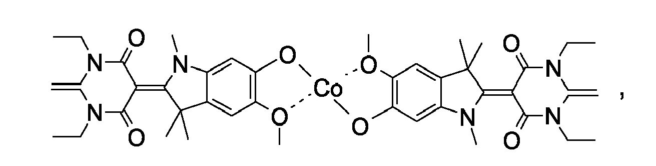

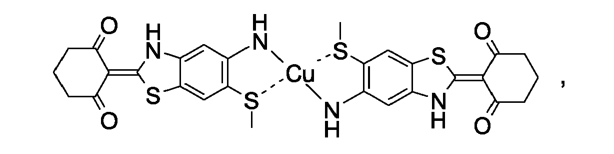

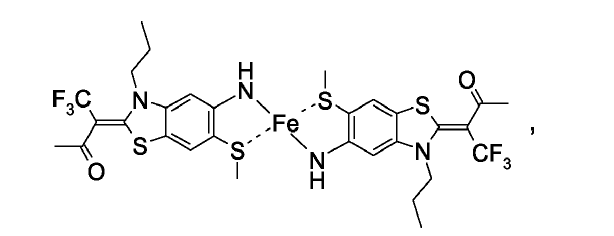

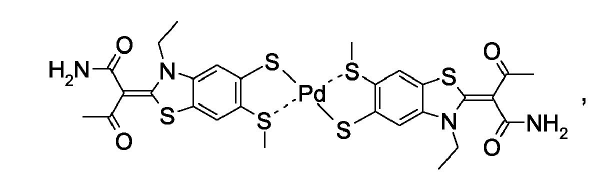

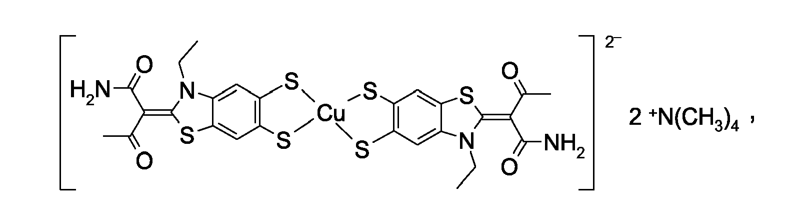

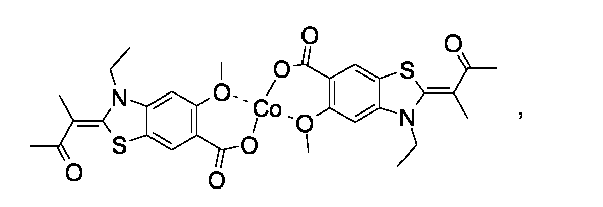

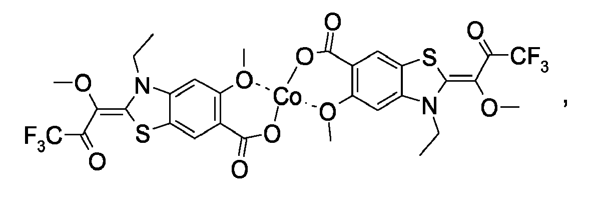

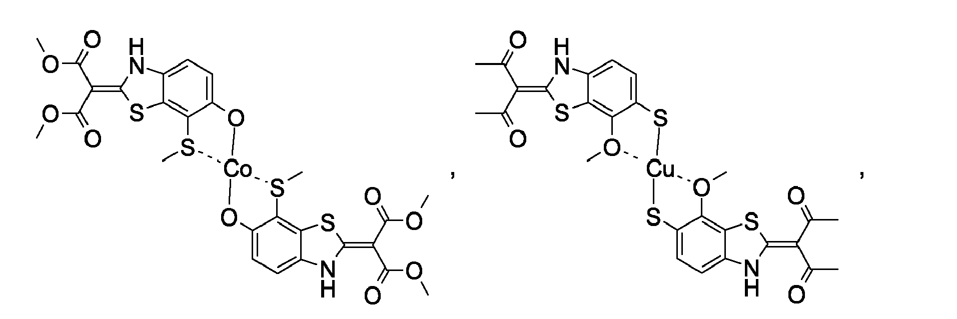

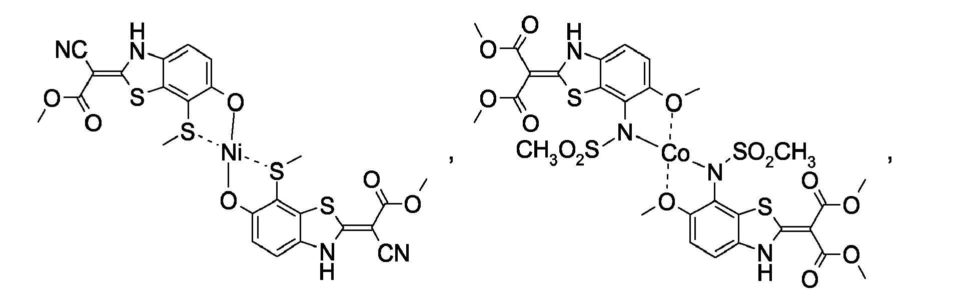

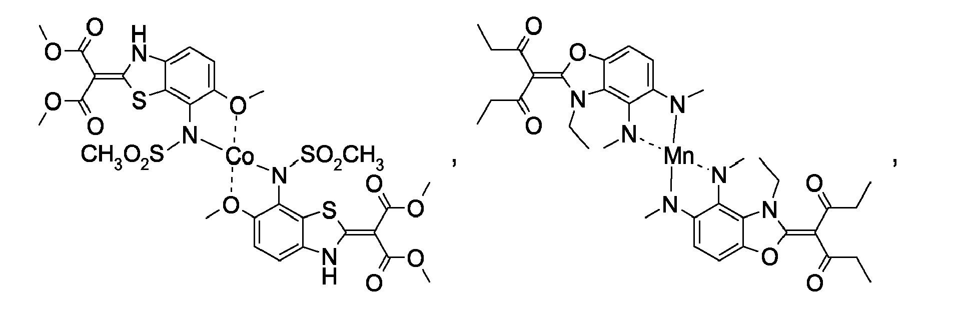

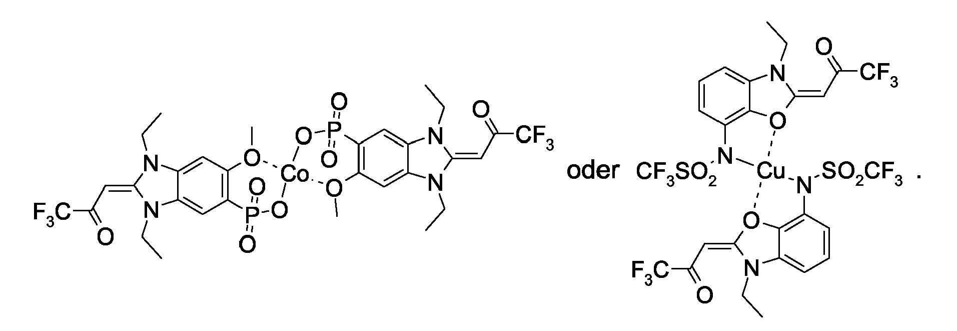

Weitere Verbindungen sind hiernach als ausführliche Beispiele aufgeführt. Selbstverständlich können am Metall auch weitere Liganden vorhanden sein, welche meist nicht sehr stark gebunden sind und daher reversibel entfernbar sind.

Die Aufzeichnungsschicht enthält zweckmässig eine Verbindung der Formel (I) oder ein Gemisch solcher Verbindungen als Hauptbestandteil oder zumindest wichtige Komponente, beispielsweise von 1 bis 100 Gew.-%, bevorzugt von 20 bis 100 Gew.-%, besonders bevorzugt von 50 bis 100 Gew.-%. Weitere übliche Bestandteile sind möglich, wie zum Beispiel andere Chromophore (beispielsweise solche mit einem Absorptionsmaximum bei 300 bis 1000 nm), Stabilisatoren, ¹O₂-, Triplett- oder Lumineszenzlöscher ("quencher"), Schmelzpunkterniedriger, Zersetzungsbeschleuniger oder alle andere Additive, welche in optischen Aufzeichnungsmitteln bereits beschrieben wurden. Bevorzugt werden gegebenenfalls Stabilisatoren oder Fluoreszenzlöscher zugegeben.

Chromophore, welche gegebenenfalls in der Aufzeichnungsschicht zusätzlich zu den Verbindungen der Formel (I) verwendet werden können, sind zum Beispiel Cyanine und Cyaninmetallkomplexe (US 5,958,650), Aza- und Phosphacyanine (WO 02/082438), Styrylverbindungen (US 6,103,331), Oxonolfarbstoffe (EP 0833314), Azofarbstoffe und Azometallkomplexe (JP 11/028865A), Phthalocyanine (EP 0 232 427, EP 0 337 209, EP 0 373 643, EP 0 463 550, EP 0 492 508, EP 0 509 423, EP 0511 590, EP 0 513 370, EP 0 514 799, EP 0 518 213, EP 0 519 419, EP 0 519 423, EP 0 575 816, EP 0600427, EP 0 676 751, EP 0 712 904, WO 98/14 520, WO 00/09 522, WO 02/25 648, WO 02/083 796, EP 1253 586, EP 1265 233, EP 1 271 500, WO 05/000 972), Porphyrine, Porphyrazine (EP 0822546, US 5,998,093, JP 2001/277723A, WO 03/042 990), Carbopyronine (WO 03/007 296), Dipyrromethenfarbstoffe und deren Metallchelatverbindungen (EP 0 822 544, EP 0 903 733), Xanthenfarbstoffe und deren Metallkomplexsalze (US 5,851,621, WO 03/098617, WO 03/098618), Pyridonmetallkomplexe (WO 03/063 151) oder Quadratsäureverbindungen (EP 0 568 877), ferner auch Oxazine, Dioxazine, Diazastyryle, Formazane, Anthrachinone oder Phenothiazine, wobei diese Liste keineswegs abschliessend ist und der Fachmann weitere bekannte Farbstoffe mitliest, zum Beispiel diejenigen aus WO 04/088649 oder PCT/EP05/050673.

Weitere, bevorzugte Chromophore, welche gegebenenfalls in der Aufzeichnungsschicht zusätzlich zu den Verbindungen der Formel (I) verwendet werden können, sind bekannte UV-Absorber, wie beispielsweise Azacyanine (JP H11/34 500), Merocyanine (WO 02/080161), Triazine (JP 2001/277720, JP 2002/160452, WO 04/106 311, JP 2004/160 883), Salicylaldehyde (JP 2004/034 645), Stilbene (JP 2003/246142), andere substituierte Olefine (US 2004/0290401) oder Metallchelate (WO 04/079732, WO 05/012 228).

Gemische weisen bekanntlich eine Reihe von Vorteilen auf, zum Beispiel eine bessere Löslichkeit und eine geringere Kristallisationstendenz, so dass es einfacher ist, durch Schleuderbeschichtung stabil amorphe Schichten herzustellen. Durch Optimierung der Mischungsverhältnisse in an sich bekannter Weise erhält man feste Aufzeichnungsschichten mit vorteilhaft thermischen und optischen Eigenschaften, insbesondere mit steilen Absorptionsbanden. Zudem kann dadurch oft der Verflachung der spektralen Absorptionskante im Festzustand entgegen gewirkt werden. Optimale Mischungsverhältnisse werden daher in der Regel durch Reihenversuche ermittelt, wobei auch verschiedene Rillengeometrien mit einbezogen werden.

Bevorzugt sind gegebenenfalls selbstverständlich solche zusätzliche Farbstoffe, welche für die Verwendung in optischen Aufzeichnungsmaterialien bei 300 bis 500 nm selbst bekannt sind. Besonders bevorzugt sind Mischungen der Verbindungen der Formel (I), beispielsweise Homologengemische und Mischungen enthaltend sowohl Verbindungen der Formel (I), worin L₁, L₂ und/oder L₃ identisch sind, als auch Verbindungen der Formel (I), worin L₁, L₂ und/oder L₃ verschieden sind, wie zum Beispiel M⁺(L₁)⁻, M⁺(X⁺)(L₁)⁻(L₁)⁻, M⁺(X⁺)(L₁)²⁻, M+(X⁺)(L₁)⁻(L₂)⁻, M+(X²⁺)(L₁)⁻(L₁)⁻(L₁)⁻, M⁺(X²⁺)(L₁)⁻(L₁)⁻(L₃)⁻,M⁺(X²⁺)(L₁)⁻(L₂)⁻(L₃)⁻, M²⁺(X⁻)(L₁)⁻, M²⁺(L₁)²⁻, M²⁺(L₁)⁻(L₁)⁻, M²⁺(L₁)⁻(L₂)⁻, M²⁺(X⁺)(L₁)⁻(L₁)⁻(L₁)⁻, M²⁺(X⁺)(L1)⁻(L₁)⁻(L₃)⁻, M²⁺(X⁺)(L₁)⁻(L₂)⁻(L₃)⁻,M²⁺(X²+)(L₁)²⁻(L₁)²⁻, M²⁺(X²+)(L₁)²⁻(L₂)²⁻,M³⁺ M³⁺(X⁻)(L₁)⁻(L₂)⁻, M³⁺(X⁻)₂(L₁)⁻, M³⁺(L₁)⁻(L₂)²⁻, M³⁺(L₁)⁻(L₁)⁻(L₁)⁻, M³⁺(L₁)⁻(L₁)⁻(L2)⁻, M³⁺(L₁) ⁻(L₂)⁻(L₃)⁻, M³⁺(X⁺)(L₁)²⁻(L₁)²⁻, M³⁺(X⁺)(L₁)²⁻(L₂)²⁻,M³⁺(X²⁺)(L₁)²⁻(L₁)²⁻(L₃)⁻ oder M³⁺(X²⁺)(L₁)²⁻(L₂)²⁻(L₃)⁻, je entweder mit zusätzlichen, neutralen und/oder anionischen Liganden oder auch ohne zusätzliche Liganden. Asymmetrische Verbindungen der Formel (I) sowie Gemische enthaltend asymmetrische Verbindungen der Formel (I) weisen in der Regel vorteilhaft eine überraschend niedrigere Kristallisationstendenz auf.

Enthält die Aufzeichnungsschicht sonstige, zur Verwendung bei 300 bis 500 nm an sich nicht geeignete Chromophore, so soll bevorzugt die Menge dieser Chromophore klein sein, so dass desser Absorption bei der Wellenlänge des Wendepunktes (Punkt der maximalen Steigung) der Steigung der langwelligen Flanke der für die Aufzeichnung massgebenden Absorptionsbande der gesamten festen Schicht einen Bruchteil der Absorption der Verbindungen der Formel (I) in der gesamten festen Schicht bei derselben Wellenlänge beträgt, zweckmässig höchstens ⅓, bevorzugt höchstens ¹/₅, besonders bevorzugt höchstens ¹/₁₀. Das Absorptionsmaximum von Farbstoffgemischen im spektralen Bereich von 300 bis 500 nm ist bevorzugt bei einer Wellenlänge tiefer als 450 nm, besonders bevorzugt tiefer als 400 nm, insbesondere bei 340-380 nm.

Stabilisatoren, ¹O₂-, Triplett- oder Lumineszenzlöscher sind zum Beispiel Metallkomplexe von N oder S enthaltenden Enolaten, Phenolaten, Bisphenolaten, Thiolaten, Bisthiolaten oder von Azo-, Azomethin- oder Formazanfarbstoffen, wie Bis(4-dimethylaminodithiobenzil)nickel [CAS N° 38465-55-3], ® Irgalan Bordeaux EL, ® Cibafast N oder ähnliche Verbindungen, gehinderte Phenole und ihre Derivate, wie ® Cibafast AO, o-Hydroxyphenyl-triazole, -triazine oder andere UV-Absorber, wie ® Cibafast W oder ®Cibafast P oder gehinderte Amine (TEMPO oder HALS, auch als Nitroxide oder NOR-HALS, ferner Dümmonium-, Paraquat™- oder Orthoquat™-Salze, wie ® Kayasorb IRG 022, ® Kayasorb IRG 040, oder gegebenenfalls auch Radikalsalze, wie N,N,N',N'-Tetrakis(4-dibutylaminophenyl)-p-phenylenamin-ammoniumsalze. Die letzteren sind von Organica (Wolfen / DE), ® Kayasorb-Marken von Nippon Kayaku Co., Ltd. und ® Irgalan- sowie ® Cibafast-Marken von Ciba Spezialitätenchemie AG erhältlich.

Viele solche Strukturen sind bekannt, teilweise auch in Zusammenhang mit optischen Aufzeichnungsmedien, beispielsweise aus US 5,219,707, JP 06 / 199 045 A, JP 07 / 76169 A, JP 07 / 262 604 A oder JP 2000/272241 A. Es kann sich dabei zum Beispiel um Salze von Metallkomplexanionen, wie aus zuvor genannten Publikationen bekannt, oder auch um Metallkomplexe handeln, illustriert zum Beispiel durch die Verbindungen der Formel

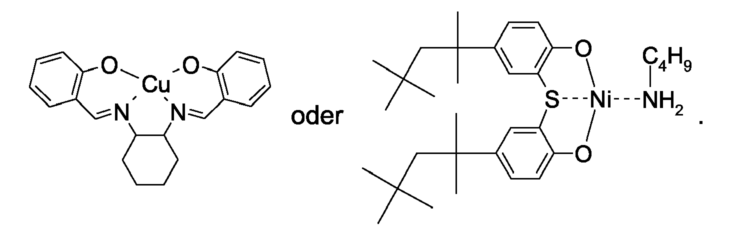

Der Fachmann weiss aus anderen optischen Informationsmedien oder wird aufgrund seines allgemeines Wissens sowie des Stands der Technik ohne Mühe erkennen, welche Additive in welcher Konzentration für welchen Zweck besonders gut geeignet sind. Geeignete Konzentrationen von Additiven sind zum Beispiel von 0,001 bis 1000 Gew.-%, bevorzugt von 1 bis 50 Gew.-%, bezogen auf das Aufzeichnungsmittel der Formel (I).

Die erfindungsgemässen optischen Aufzeichnungsmaterialien weisen insgesamt hervorragende spektrale Eigenschaften der festen, amorphen Aufzeichnungsschicht auf, wobei auch der Brechungsindex überraschend hoch ist. Dank einer für solche Verbindungen überraschend geringen Aggregationstendenz im Feststoff ist die Absorptionsbande schmal und intensiv, mit besonders hoher Steilheit der Absorptionsbande auf der langwelligen Seite. Kristallite werden unerwartet und vorteilhaft nicht oder nur in vernachlässigbarem Mass gebildet. Die Reflektivität der Schichten im Bereich der Schreib- und Lesewellenlänge ist im unbeschrifteten Zustand hoch. Die Empfindlichkeit gegenüber Laserstrahlung ist im Schreibmodus hoch; im energieärmeren Auslesemodus ist die Stabilität dagegen hoch.

Dank diesen hervorragenden Schichteigenschaften ist eine schnelle optische Aufzeichnung mit hoher Empfindlichkeit, hoher Reproduzierbarkeit und geometrisch sehr genauen Markenabgrenzungen möglich, wobei sich der Brechungsindex und die Reflektivität stark ändern, was einen hohen Kontrast ergibt. Die Abweichungen der Markenlängen und Intervalldistanzen ("jitter") sind sowohl bei normaler (zirka 4,5 bis 5,5 m · s⁻¹) als auch bei höherer Aufnahmegeschwindigkeit (zum Beispiel von 9 m · s⁻¹ bis 25 m · s⁻¹ oder sogar höher) überraschend klein. Das ermöglicht eine hohe Speicherdichte durch einen schmalen Aufzeichnungskanal mit geringerem Spurabstand ("pitch"). Zudem werden die aufgezeichneten Daten mit erstaunlich kleiner Fehlerrate wiedergegeben, so dass kürzere Marken möglich sind, darunter zum Beispiel solche der Länge 0,15±0,01 µm (2T) im Einklang mit dem Blu-ray™ Standard, und die Fehlerkorrektur nur wenig Speicherplatz erfordert.

Dank der ausgezeichneten Löslichkeit, teilweise auch in apolaren Lösungsmitteln, können Lösungen auch mit hoher Konzentration ohne störende Ausfällungen beispielsweise beim Lagern verwendet werden, so dass Probleme bei der Aufschleuderung weitgehend entfallen. Dies gilt insbesondere für Verbindungen enthaltend verzweigtes C₃-C₅Alkyl.

Aufnahme und Wiedergabe können bei der gleichen Wellenlänge mit einer Laserquelle von zweckmässig 300 bis 500 nm, insbesondere 350 bis 500 nm, bevorzugt 370 bis 450 nm erfolgen. Besonders bevorzugt sind der UV-Bereich von 370 bis 390 nm, insbesondere etwa 380 nm, sowie hauptsächlich am Rande des sichtbaren Bereiches von 390 bis 430 nm, speziell etwa 405±5 nm. Im Bereich von kompakten, blauen oder violetten Laserdioden (wie Nichia GaN 405 nm) mit Optik hoher numerischer Apertur (beispielsweise 0,85) können die Marken so klein und die Spuren so eng dimensioniert sein, dass etwa 20 bis 30 Gb pro Aufzeichnungsschicht auf einer 120 mm Scheibe ("disc") erreichbar sind. Bei 380 nm kann man etwa mit Indium dotierten UV-VCSELs (Vertical-Cavity Surface-Emitting Laser) verwenden (Jung Han et al., vgl. MRS Internet J. Nitride Semicond. Res. 5S1, W6.2 [2000]).

Die Erfindung betrifft daher auch ein Verfahren zur Aufzeichnung oder Wiedergabe von Daten, dadurch gekennzeichnet, dass die Daten auf einem erfindungsgemässen optischen Aufzeichnungsmedium bei einer Wellenlänge von 300 bis 500 nm aufgezeichnet oder wiedergegeben werden. Die Aufzeichnung findet bevorzugt bei einer linearen Geschwindigkeit *v* von mindestens 4,5 m·s⁻¹ statt, wobei besonders bevorzugt Marken verschiedener Länge erzeugt werden, wovon die kürzesten fast kreisrund sind und die längsten eine Länge aufweisen, welche etwa der vierfachen Breite entspricht. Die lineare Geschwindigkeit beträgt besonders bevorzugt mindestens 9 m·s⁻¹ (1×), 18 m·s⁻¹ (2×) oder 36 m·s⁻¹ (4×).

Das Aufzeichnungsmedium kann sich am Aufbau bekannter Aufzeichnungsmedien anlehnen und ist dann zum Beispiel denjenigen analog, welche zuvor erwähnt wurden, wie DVD+R oder DVD-R. Es kann daher beispielsweise aus einem transparenten Substrat, einer Aufzeichnungsschicht, enthaltend mindestens eine der Verbindungen der Formel (I), einer Reflektorschicht und einer Deckschicht aufgebaut sein, wobei das Einschreiben und Auslesen durch das Substrat erfolgt. Ein solches, für Aufnahme und Wiedergabe bei einer Wellenlänge von 300 bis 500 nm geeignetes System ist beispielsweise HD DVD™ (ehemals advanced optical disk AOD genannt).

Geeignete Substrate sind zum Beispiel Gläser, Mineralien, Keramiken und duroplastische oder thermoplastische Kunststoffe. Bevorzugte Träger sind Gläser und homo- oder copolymere Kunststoffe. Geeignete Kunststoffe sind zum Beispiel thermoplastische Polycarbonate, Polyamide, Polyester, Polyacrylate und Polymethacrylate, Polyurethane, Polyolefine, Polyvinylchlorid, Polyvinylidenfluorid, Polyimide, duroplastische Polyester und Epoxidharze. Besonders bevorzugt sind Polycarbonat-Substrate, die beispielsweise mittels Einspritzverfahren ("injectionmoulding") herstellbar sind. Das Substrat kann in reiner Form sein oder auch übliche Additive enthalten, beispielsweise UV-Absorber oder Farbstoffe, wie zum Beispiel in JP 04/167239A als Lichtschutz für die Aufzeichnungsschicht vorgeschlagen wird. In letzterem Fall ist es gegebenenfalls günstig, dass der zum Trägersubstrat zugesetzte Farbstoff im Bereich der Einschreib-Wellenlänge (Emissionswellenlänge des Lasers) keine oder höchstens eine geringe Absorption aufweist, bevorzugt bis maximal etwa 20% des auf die Aufzeichnungsschicht fokussierten Laserlichtes.

Zweckmässig ist das Substrat in mindestens einem Teil des Bereichs von 300 bis 500 nm transparent, so dass es für beispielsweise mindestens 80% des darauf fallenden Lichtes der Einschreib- oder Auslesewellenlänge durchlässig ist. Das Substrat weist zweckmässig eine Dicke von 10 µm bis 2 mm, bevorzugt von 100 bis 1200 µm, besonders bevorzugt von 600 bis 1100 µm auf, mit einer bevorzugt spiralförmigen Führungsrille (Spur) auf der Beschichtungsseite, mit einer Rillentiefe von 10 bis 200 nm, bevorzugt von 60 bis 150 nm, einer Rillenbreite von 100 bis 400 nm, bevorzugt von 150 bis 250 nm und einem Achsenabstand zwischen 2 Rillen von 200 bis 600 nm, bevorzugt von 250 bis 450 nm (beispielsweise mit Rillentiefe von 80±20 nm, Rillenbreite 200±50 nm und Achsenabstand zwischen 2 Windungen 370±60 nm). Rillen verschiedener Querschnittprofile sind bekannt, zum Beispiel rechteckige, trapez- oder V-förmige. Analog zu den bekannten CD-R und DVD±R Medien kann die Führungsrille zusätzlich eine kleine periodische oder quasiperiodische seitliche Auslenkung ("wobble") erfahren, wodurch die Synchronisation der Drehzahl und die absolute Positionierung des Auslesekopfs ("pick-up") ermöglicht wird. Dieselbe Funktion kann anstelle der Auslenkung oder zusätzlich durch Markierungen zwischen benachbarten Rillen erfolgen ("pre-pits").

Das Aufzeichnungsmittel wird beispielsweise durch Aufschleuderung einer Lösung aufgetragen, wobei eine möglichst amorphe Schicht entstehen soll, deren Dicke in der Rille abhängig von der Rillengeometrie zweckmässig von 20 bis 150 nm, bevorzugt von 30 bis 120 nm, besonders bevorzugt von 30 bis 80 nm und nebenan ("land") zweckmässig von 0 bis 70 nm, bevorzugt von 1 bis 20 nm, besonders bevorzugt von 2 bis 10 nm und betragen. In einer anderen, mit den Verbindungen der Formel (I) realisierbaren Ausführungsform kann die Dicke der Aufzeichnungsschicht zweckmässig in der Rille 30 bis 80 nm und nebenan ("land") 20 bis 70 nm betragen, wobei die Differenz der Schichtdicken in der Rille und auf der Oberfläche weniger als 20 nm, bevorzugt weniger als 10 nm beträgt. Dadurch ist es möglich, mit HD DVD Rewritable™ kompatibel sowohl in den Rillen als auch auf der Oberfläche nebenbei zu schreiben und lesen. Der track pitch ist dann nur etwa halb so gross, und die gesamte Speicherkapazität ist höher.

In beiden Ausführungsformen erfolgt das Einschreiben und Auslesen von der Substratseite her. Der Laserstrahl wird dabei durch das Substrat auf die Aufzeichnungsschicht gerichtet und weist bevorzugt eine Wellenlänge von 300 bis 500 nm, besonders bevorzugt von 370 bis 450 nm auf. Auf der dem Substrat entgegensesetzten Seite der Aufzeichnungsschicht befindet sich gegebenenfalls eine Reflektorschicht.

Als reflektierendes Material für die Reflektorschicht eignen sich besonders Metalle, welche die zur Aufzeichnung und Wiedergabe verwendete Laserstrahlung gut reflektieren, zum Beispiel die Metalle der dritten, vierten und fünften Hauptgruppe und der Nebengruppen des periodischen Systems der chemischen Elemente. Besonders geeignet sind Al, In, Sn, Pb, Sb, Bi, Cu, Ag, Au, Zn, Cd, Hg, Sc, Y, La, Ti, Zr, Hf, V, Nb, Ta, Cr, Mo, W, Fe, Co, Ni, Ru, Rh, Pd, Os, Ir, Pt und die Lanthanidenmetalle Ce, Pr, Nd, Pm, Sm, Eu, Gd, Tb, Dy, Ho, Er, Tm, Yb und Lu, sowie deren Legierungen. Besonders bevorzugt ist aus Gründen der hohen Reflektivität und leichten Herstellbarkeit eine reflektierende Schicht aus Aluminium, Silber, Gold oder eine Legierung davon (beispielsweise eine Weissgold- oder Silber/Chrom-Legierung), aus ökonomischen und ökologischen Gründen insbesondere Aluminium. Die Reflektorschicht hat eine Dicke zweckmässig von 5 bis 200 nm, bevorzugt von 10 bis 100 nm, besonders bevorzugt von 20 bis 80 nm, wobei auch dickere Reflektorschichten möglich sind.

Als Material für die Deckschicht eignen sich hauptsächlich Kunststoffe, die in dünner Schicht entweder direkt oder mit Hilfe von Haftvermittlern auf die Reflektorschicht aufgebracht werden. Man wählt zweckmässig mechanisch und thermisch stabile Kunststoffe mit guten Oberflächeneigenschaften, die noch modifiziert, zum Beispiel beschrieben werden können. Es kann sich sowohl um duroplastische wie auch um thermoplastische Kunststoffe handeln. Bevorzugt für direkt aufgebrachte Deckschichten sind strahlungsgehärtete (zum Beispiel mit UV-Strahlung) Beschichtungen, die besonders einfach und wirtschaftlich herstellbar sind. Strahlungshärtbare Materialien sind in grosser Vielzahl bekannt. Beispiele für strahlungshärtbare Monomere und Oligomere sind Acrylate und Methacrylate von Diolen, Triolen und Tetrolen, Polyimide aus aromatischen Tetracarbonsäuren und aromatischen Diaminen mit C₁-C₄Alkylgruppen in mindestens zwei Orthostellungen der Aminogruppen, und Oligomere mit Dialkyl-, zum Beispiel Dimethylmaleinimidylgruppen. Bevorzugt für durch Haftvermittler aufgebrachte Deckschichten werden die gleichen Materialien wie für die Substratschicht verwendet, besonders bevorzugt Polycarbonate. Als Haftvermittler werden bevorzugt ebenfalls strahlungshärtbare Monomere und Oligomere verwendet. Anstelle der durch einen Haftvermittler aufgebrachten Deckschicht kann auch ein zweites, eine Aufzeichnungs- und Reflektorschicht enthaltendes Substrat verwendet werden, so dass das Aufzeichnungsmedium beidseitig bespielbar ist. Bevorzugt ist ein symmetrischer Aufbau, wobei die beiden Teile reflektorseitig durch einen Haftvermittler direkt oder über eine Zwischenschicht zusammengefügt werden. In diesem Fall ist das Substrat selbstverständlich nur etwa halb so dick, so dass die gesamte, aus zwei Substraten zusammengesetzte Disk etwa gleich dick ist, wie eine aus nur einem Substrat bestehende Disk.

Bei dieser Aufbauweise spielen an sich die optischen Eigenschaften der Deckschicht, beziehungsweise der Deckmaterialien, im wesentlichen keine Rolle, sofern gegebenenfalls deren Härtung beipielsweise durch UV-Strahlung gewährleistet ist. Die Funktion der Deckschicht ist, die mechanische Festigkeit des Aufzeichnungsmediums als Ganzes sowie wenn nötig die mechanische Festigkeit dünner Reflektorschichten zu gewährleisten. Bei genügend stabilem Aufzeichnungsmedium, zum Beispiel in Anwesenheit einer dicken Reflektorschicht kann man daher sogar auf die Deckschicht ganz verzichten. Die Dicke der Deckschicht hängt von der Dicke des gesamten Aufzeichnungsmediums ab, welches bevorzugt maximal etwa 2 mm dick sein sollte. Bevorzugt weist die Deckschicht ein Dicke von 10 µm bis 1 mm auf.

Die erfindungsgemässen Aufzeichnungsmedien können auch zusätzliche Schichten aufweisen, wie zum Beispiel Interferenzschichten oder Barrierenschichten. Es ist auch möglich, Aufzeichnungsmedien mit mehreren (zum Beispiel 2 bis 10) Aufzeichnungsschichten aufzubauen. Der Aufbau und die Verwendung solcher Materialen sind dem Fachmann bekannt. Bevorzugt sind gegebenenfalls Interferenzschichten, welche zwischen der Aufzeichnungsschicht und der reflektierenden Schicht, zwischen der Aufzeichnungsschicht und dem Substrat und/oder insbesondere zwischen der Aufzeichnungsschicht und der Schutzschicht angeordnet sind und aus einem dielektrischen Material bestehen, zum Beispiel wie in EP 0 353 393 beschrieben aus TiO₂, Si₃N₄, ZnS oder Silikonharzen.

Die erfindungsgemässen Aufzeichnungsmedien können nach an sich bekannten Verfahren hergestellt werden, wobei man je nach verwendeten Materialien und deren Funktionsweise unterschiedliche Beschichtungsmethoden anwenden kann.

Geeignete Beschichtungsverfahren sind zum Beispiel Tauchen, Giessen, Streichen, Rakeln und Schleudergiessen, sowie Aufdampfverfahren, die im Hochvakuum durchgeführt werden. Bei der Anwendung von zum Beispiel Giessverfahren werden im allgemeinen Lösungen in organischen Lösungsmitteln verwendet. Bei der Verwendung von Lösungsmitteln ist darauf zu achten, dass die verwendeten Träger gegen diese Lösungsmittel unempfindlich sind. Geeignete Beschichtungsverfahren und Lösungsmittel sind zum Beispiel in EP 0401 791 oder EP 0 485 337 beschrieben.

Die Aufzeichnungsschicht wird bevorzugt durch Aufschleudern einer Farbstofflösung aufgebracht, wobei als Lösungsmittel insbesondere Alkohole, wie zum Beispiel 2-Methoxyethanol, 1-Methoxy-2-propanol, 2-Propanol oder n-Butanol, Hydroxyketone, wie zum Beispiel Diacetonalkohol oder 3-Hydroxy-3-methyl-2-butanon, Hydroxyester, wie zum Beispiel Milchsäuremethylester oder Isobuttersäuremethylester, oder bevorzugt fluorierte Alkohole, wie zum Beispiel 2,2,2-Trifluorethanol oder 2,2,3,3-Tetrafluor-1-propanol, und Gemische davon, sich bewährt haben. Weitere geeignete Lösungsmittel sind beispielsweise in EP 0483387 offenbart.

Das Aufbringen der metallischen Reflektorschicht erfolgt bevorzugt durch Zerstäuben ("sputtern") oder Aufdampfen im Vakuum. Diese Techniken sind bekannt und in Fachbüchern beschrieben (z.B. J.L. Vossen und W. Kern, "Thin Film Processes", Academic Press, 1978). Man kann vorteilhaft kontinuierlich arbeiten und erreicht eine gute Reflektivität sowie eine hohe Haftfähigkeit der metallischen Reflektorschicht.

Die Aufzeichnung erfolgt nach bekannten Verfahren durch Einschreiben von Pits (Marken) fester oder meist variabler Länge mittels eines modulierten, fokussierten und mit konstanter oder variabler Geschwindigkeit auf der Fläche der Aufzeichnungsschicht geführten Laserstrahls. Das Auslesen der Information erfolgt nach an sich bekannten Methoden durch Registrierung der Veränderung der Reflexion unter Verwendung von Laserstrahlung, zum Beispiel wie in "CD-Player und R-DAT Recorder" (Claus Biaesch-Wiepke, Vogel Buchverlag, Würzburg 1992) beschrieben. Die Anforderungen sind dem Fachmann bekannt.

Das erfindungsgemässe Informationen enthaltende Medium stellt insbesondere ein optisches Informationsmaterial vom WORM-Typ dar. Es kann zum Beispiel analog zur CD-R (compact disc - recordable) oder DVD±R (digital video disc - recordable) in Rechnern verwendet werden, sowie auch als Speichermaterial für Ausweis- und Sicherheitskarten oder für die Herstellung von diffraktiven optischen Elementen, beispielsweise Hologrammen. Aufzeichnungsmedien des Typs HD DVD™ erlauben die Verwendung eines Lasers mit einer numerischen Apertur von maximal etwa 0,7 (üblicherweise 0,60 bis 0,65). Bei einer Aufnahmegeschwindigkeit von 6,61 m·s⁻¹ (oder eines Mehrfaches davon) ergibt sich dabei für Disks mit 120 mm Durchmesser eine Speicherkapazität von 15 GB pro Aufzeichnungsschicht.

Es gibt aber alternativ auch neuere Aufzeichnungsmedien, welche sich von CD-R oder DVD ± R stark unterscheiden, und worin Aufzeichnung und Wiedergabe nicht durch das Substrat, sondern durch die Deckschicht erfolgen. Somit sind die respektiven Rollen der Deckschicht und des Substrats, insbesondere die Geometrie und die optischen Eigenschaften, im Vergleich zum zuvor beschriebenen Aufbau vertauscht. Entsprechende Konzepte für digitale Videoaufnahmen in Verbund mit einer blauen GaN Laserdiode sind bekannt, zum Beispiel aus Proceedings SPIE-Int. Soc. Opt. Eng. 1999, 3864. In fortgeschrittener Entwicklung befindet sich auch Blu-ray™(ehemals Blu-ray Disk "BD") mit einer Aufnahmegeschwindigkeit von 5,0±0,3 m·s⁻¹ (voraussichtlich bald ein Mehrfaches davon) und einer Speicherkapazität von 25±2 GB (vgl. Systembeschreibung "Blu-ray Disc Rewritable Format version 1.0" / Juni 2002 sowie Blu-ray.com).

Die erfindungsgemässen Verbindungen der Formel (I) kommen aber auch diesen erhöhten Anforderungen beim inversen Schichtaufbau überraschend gut entgegen. Bevorzugt ist daher ein inverser Schichtaufbau, mit der Schichtabfolge Substrat, Reflektorschicht, Aufzeichnungsschicht und Deckschicht. Die Aufzeichnungsschicht befindet sich also zwischen der Reflektorschicht und der Deckschicht. Eine etwa 50 bis 400 µm dünne Deckschicht ist besonders zweckmässig (typischerweise 100 µm bei einer numerischen Apertur von 0,85).

Aufzeichnungs- und Reflektorschichten haben beim inversen Schichtaufbau prinzipiell die gleiche Funktion, wie zuvor angegeben. Das Substrat weist üblicherweise Dimensionen innerhalb der zuvor angegebenen Bereichen auf. Zweckmässig hat die bevorzugt spiralförmige Führungsrille (Spur) auf der Beschichtungsseite eine Rillentiefe von 10 bis 100 nm, bevorzugt 20 bis 80 nm. Querschnittprofil, periodische oder quasiperiodische seitliche Auslenkung ("wobble") sowie gegebenenfalls zusätzliche Markierungen zwischen benachbarten Rillen erfolgen ("pre-pits") lehnen sich dem zuvor beschriebenen HD DVD™Typ an.

Auf dem Substrat werden Reflektorschicht und Aufnahmeschicht in dieser Reihenfolge aufgetragen. Als Spur können sowohl die Rillen als auch die sich dazwischen erhebenden, schienenartigen Zinnen benützt werden, wobei man üblicherweise im ersten Fall von "in-groove" und im zweiten Fall von "on-groove" Medien spricht. Mit den Verbindungen der Formel (I) sind vorteilhaft beide Ausführungsformen realisierbar, gegebenenfalls auch simultan.

Das Aufzeichnungsmittel wird beispielsweise wie zuvor angegeben aufgetragen. Vorteilhaft ist dabei insbesondere, dass man auch Lösungsmittel wählen kann, welche das Substratmaterial angreifen würden, zum Beispiel chlorierte oder aromatische Kohlenwasserstoffe. Die Dicke der möglichst amorphen Schicht kann gleichmässig oder in den Rillen und auf den Zinnen unterschiedlich sein. In den Rillen beträgt die Dicke der Aufzeichnungsschicht zweckmässig von 20 bis 200 nm, bevorzugt von 30 bis 150 nm, besonders bevorzugt von 30 bis 100 nm. Soll die Spur auf den Zinnen zur Aufzeichnung verwendet werden, beträgt deren Schichtdicke zweckmässig von 10 bis 120 nm, bevorzugt von 20 bis 100 nm, besonders bevorzugt von 20 bis 60 nm. Wird hingegen nur die Rille als Spur benützt, genügt eine Schichtdicke von 0 bis 100 nm, bevorzugt von 0 bis 60 nm, besonders bevorzugt von 0 bis 20 nm. In beiden Fällen beträgt die Spurbreite (Zinnen und/oder Kerben) von 100 bis 300 nm, bevorzugt von 120 bis 250 nm, besonders bevorzugt von 150 bis 200 nm, und der Achsenabstand zwischen 2 Spuren von 200 bis 600 nm, bevorzugt von 250 bis 400 nm, besonders bevorzugt von 300 bis 340 nm. Gute Resultate bekommt man beispielsweise mit einer 30± 10 nm tiefen und 180± 10 nm breiten Zinnenspur ("on-groove") mit einem Achsenabstand von 320± 10 nm). Der Laserstrahl geht dabei mit einer hohe Apertur durch die Deckschicht, was die Auflösung erhöht.

Der inverse Schichtaufbau stellt aber wesentlich höhere Ansprüche, welchen die erfindungsgemäss verwendeten Verbindungen erstaunlich gut genügen. Besonders hohe Ansprüche stellen sich beispielsweise beim Aufbringen der Aufzeichnungsschicht auf die metallische Reflektorschicht sowie insbesondere beim Aufbringen einer Deckschicht auf die Aufzeichnungsschicht, welche letztere ausreichend vor Reibung, Photooxydation, Fingerabdrücken, Feuchtigkeit und anderen Umwelteinflüssen schützen soll und zweckmässig eine Dicke im Bereich von 0,01 bis 0,5 mm, vorzugsweise im Bereich von 0,05 bis 0,2 mm, besonders bevorzugt im Bereich von 0,08 bis 0,13 mm aufweist.

Die Deckschicht besteht bevorzugt aus einem Material, das eine Transmission von 80% oder höher bei der Einschreib-oder Auslesewellenlänge des Lasers zeigt. Als Material für die Deckschicht eignen sich bespielsweise die zuvor angegebenen Materialien, insbesondere aber Polycarbonat (wie Pure Ace® oder Panlite® , Teijin Ltd), Cellulosetriacetat (wie Fujitac® , Fuji Photo Film) oder Polyethylenterephthalat (wie Lumirror® , Toray Industry), wobei Polycarbonat besonders bevorzugt ist. Besonders für direkt aufgebrachte Deckschichten sind strahlungsgehärtete Beschichtungen, wie bereits vorgängig beschrieben, zweckmässig, zum Beispiel SD 347™ (Dainippon Ink).

Die Deckschicht kann durch einen geeigneten Haftvermittler direkt auf die feste Aufzeichnungsschicht aufgebracht werden. In einer anderen Ausführungsform wird auf die feste Aufzeichnungsschicht eine zusätzliche, dünne Trennschicht aus einem metallischen, vernetzten metallorganischen oder bevorzugt dielektrischen anorganischen Stoff aufgebracht, zum Beispiel in einer Dicke von 0,001 bis 10 µm, bevorzugt von 0,005 bis 1 µm, besonders bevorzugt von 0,01 bis 0,1 µm, beispielsweise von 0,05 bis 0,08 µm für dielektrische und von 0,01 bis 0,03 µm für metallische Trennschichten. Trennschichten sowie entsprechende Verfahren sind in WO 02/082438 offenbart, worauf hier ausdrücklich verwiesen sei. Falls erwünscht, können solche Beschichtungen zum Beispiel in gleicher Dicke auch zwischen dem Trägermaterial und der metallischen Reflektorschicht oder zwischen der metallischen Reflektorschicht und der optischen Aufzeichnungsschicht angebracht werden. Dies kann in gewissen Fällen Vorteile bringen, zum Beispiel bei Verwendung eines Silber-Reflektors in Kombination mit schwefelhaltigen Additiven in der Aufzeichnungsschicht.

Analog zum vorher beschriebenen Aufbau können selbstverständlich auch hier diese Aufzeichnungsmedien aus zwei Hälften zusammengefügt werden, wobei zwei Substrate vor oder nach der Beschichtung geklebt werden. Es ist zudem auch möglich, ein Substrat zu verwenden, welches auf beiden Seiten gerillt ist.

Ein ganz besonderer Vorteil der erfindungsgemässen Verbindungen sind ihre Leistungswerte in dem Blu-ray™ Standard entsprechenden Aufzeichnungsmedien. Die erfindungsgemässen Verbindungen können dementsprechend auch zur technischen Verbesserung von optischen Aufzeichnungsmitteln verwendet werden.

Bei einem Aufzeichnungsmedium mit inversem Schichtaufbau spielt andererseits die Transparenz des Substrates keine Rolle. Es ist also auch möglich, beispielsweise gefärbte (zum Beispiel gelb, rot, blau, grün, blau, weiss, grau oder schwarz pigmentierte) Kunststoffe zu verwenden, oder auch andere synthetische oder natürliche Materialien, wie Stahl-, Aluminium- oder andere Metalle, oder auch Papier (vgl. Proceedings of SPIE Vol. 5380 / 04 "A 25GB paper disk").

Die erfindungsgemässen Verbindungen der Formel (I) sind neu. Die Erfindung betrifft daher auch eine Verbindung der Formel (I) gemäss der zuvor angegebenen Definition.

Die Verbindungen der Formel (I) werden zweckmässig durch Umsetzung von Liganden mit Metallsalzen analog zu an sich bekannten Methoden hergestellt. Brauchbar ist zum Beispiel das Verfahren, welches in WO 05/000 972 offenbart ist.

Zusätzliche Liganden sind gegebenenfalls zweckmässig in 1,0 bis 1,2-facher stöchiometrischer Menge vorhanden und werden bevorzugt erst in der letzten Herstellungsstufe zugegeben. Die stöchiometrische Menge entspricht der gewünschten Anzahl solcher Liganden im Chelat der Formel (I).

Gegebenenfalls kann die Ausfällung des gewünschten Produktes begünstigt oder beschleunigt werden, indem die Mutterlauge verdünnt wird. Verdünnungsmittel können je nach Reaktionsflüssigkeit und Polarität des gewünschten Produktes nach üblichen, an sich bekannten Kriterien ausgewählt werden, oft sind Wasser oder apolare Kohlenwasserstoffe geeignet.

Die Isolierung der Chelate geschieht in der Regel durch Filtration oder durch Extraktion aus wässriger Phase (gegebenenfalls nach Zugabe von Wasser) mittels eines mit Wasser nicht mischbaren Lösemittels. Isolierung von Substanzen durch Ausschütteln sind inklusiv allen darauffolgenden Schritten an sich gut bekannt. Es können aber auch beliebige alternative Verfahren benützt werden, zum Beispiel Flash-Chromatographie.

Die nachfolgenden Beispiele erläutern die Erfindung, ohne deren Umfang einzuschränken (wo nicht anders angegeben, handelt es sich bei "%" immer um Gewichts-%); die Stereochemie der Doppelbindung (E/Z) ist nicht überall gesichert.

### Beispiel 1:

Stufe 1: In einem 1 l Mehrhalskolben, versehen mit Magnetrührer, Thermometer, Tropftrichter, Rückflusskühler und Stickstoffüberleitung, werden 44,0 g 5-Hydroxy-2-hydroxymethyl-pyran-4-on in 400 ml Chloroform vorgelegt und 71,4 g Thionylchlorid zugetropft. Das Gemisch wird während 3 Std. zum Rückfluss erhitzt, dann auf 0°C abgekühlt und 30 Min. gerührt. Die Suspension wird filtriert, der Rückstand mit 50 ml eiskaltem Chloroform gewaschen und 18 Std. bei 60°C/2,5 10³ Pa getrocknet. Man erhält 40 g 2-Chlormethyl-5-hydroxy-pyran-4-on.
- ¹H-NMR (DMSO-d₆):: 9,1 ppm (breiter s, 1H); 8,1 ppm (s, 1H); 6,55 ppm (s, 1H); 4,63 ppm (s, 2H).

Stufe 2: In einem 1 l Mehrhalskolben, versehen mit Magnetrührer, Thermometer, Tropftrichter und Stickstoffüberleitung, werden 90 g 2-Chlormethyl-5-hydroxy-pyran-4-on vorgelegt und 245 ml 10%ige wässrige KOH-Lösung zugegeben. Anschliessend werden 42 ml Dimethylsulfat zugetropft und 30 Min. nachgerührt. Dann werden nochmals 245 ml 10%ige wässrige KOH-Lösung zugegeben, 42 ml Dimethylsulfat zugetropft und 15 Min. nachgerührt. Das Reaktionsgemisch wird drei Mal je mit 500 ml Chloroform extrahiert, die vereinigten organischen Phasen drei Mal mit je 300 ml Wasser gewaschen, über Natriumsulfat getrocknet, filtriert und eingedampft. Der Rückstand wird in einem Gemisch von 200 ml Chloroform und 600 ml Hexan gelöst, dann wird 4 g Aktivkohle zugegeben, 30 Min. zum Rückfluss erhitzt, filtriert und eingedampft. Man erhält 50 g 2-Chlormethyl-5-methoxy-pyran-4-on.
- ¹H-NMR (CDCl₃):: 7,58 ppm (s, 1H); 6,47 ppm (s, 1H); 4,31 ppm (s, 2H); 3,78 ppm (s, 3H).

Stufe 3: In einem 1 l Mehrhalskolben, versehen mit Magnetrührer, Thermometer, Rückflusskühler und Stickstoffüberleitung, werden 24,5 g 2-Chlormethyl-5-methoxy-pyran-4-on und 10,64 g Thioharnstoff in 420 ml 95%igem Ethanol vorgelegt und während 3 Std. unter Rückfluss erhitzt. Anschliessend werden 400 ml Ethanol abdestilliert und 18 Std. bei 23°C stehen gelassen. Die Suspension wird filtriert, der Rückstand mit Ethanol gewaschen und über Nacht bei 50°C/2,5·10³ Pa getrocknet. Man erhält 20 g 2-Amino-5-hydroxy-6-methoxy-benzthiazol hydrochlorid.
- ¹H-NMR (DMSO-d₆):: 9,61 ppm (s, 3H); 7,44 ppm (s, 1H); 7,02 ppm (s, 1H); 3,75 ppm (s, 3H); 2,49 ppm (s, 1H).

Stufe 4: In einem 1 l Mehrhalskolben, versehen mit Magnetrührer, Thermometer, Tropftrichter und Stickstoffüberleitung, werden 21 g 2-Amino-5-hydroxy-6-methoxy-benzthiazol (frisch hergestellt aus 2-Amino-5-hydroxy-6-methoxy-benzthiazol hydrochlorid durch Neutralisation mit 10%iger wässriger Natriumcarbonat-Lösung, Extraktion mit Ethylacetat und Eindampfen) in 400 ml Dichlormethan gelöst, auf 0°C gekühlt und innerhalb von 10 Min. tropfenweise mit 16,3 ml Triethylamin versetzt. Innerhalb von 20 Min. werden anschliessend 22,3 g p-Toluolsulfonsäurechlorid gelöst in 200 ml Dichlormethan zugetropft und 2 Std. bei 23°C gerührt. Anschliessend werden nochmals 2 ml Triethylamin und 3 g p-Toluolsulfonsäurechlorid zugegeben und 1 Std gerührt. Das Reaktionsgemisch wird mit 1 l Ethylacetat und 500 ml Wasser während 15 Min. verrührt, die Phasen getrennt, die organische Phase mit Wasser und gesättigter wässriger NaCl-Lösung gewaschen, über Natriumsulfat getrocknet, filtriert und eingedampft. Man erhält 35 g p-Toluolsulfonsäure-2-amino-6-methoxy-benzthiazol-5-yl ester.
- ¹H-NMR (DMSO-d₆):: 7,69 ppm (d, 2H); 7,46 - 7,41 ppm (m, 5H); 6,94 ppm (s, 1H); 3,47 ppm (s, 3H); 2,40 ppm (s, 3H).

Stufe 5: In einem 1 l Mehrhalskolben, versehen mit Magnetrührer, Thermometer, Tropftrichter, Rückflusskühler und Stickstoffüberleitung, werden 50 ml Polyethylenglykol 300 und 200 ml Acetonitril vorgelegt, 16,06 g Kupfer(II)-chlorid, 35 g p-Toluolsulfonsäure-2-amino-6-methoxy-benzothiazol-5-yl-ester zugegeben und 23,9 g Isoamylnitrit, gelöst in 50 ml Polyethylenglykol 300 und 150 ml Acetonitril, zugetropft. Das Reaktionsgemisch wird während 2 Std. auf 65°C erwärmt, auf 23°C abgekühlt, mit 700 ml Eiswasser hydrolisiert und 20 Min. gerührt. Die Suspension wird filtriert, der Rückstand mit Wasser gewaschen und an der Luft getrocknet. Das Rohprodukt wird mit einem Gemisch von 200 ml Chloroform und 800 ml Hexan zum Rückfluss erhitzt, heiss filtriert und das Filtrat eingedampft. Man erhält 28 g p-Toluolsulfonsäure-2-chlor-6-methoxy-benzthiazol-5-yl ester.
- ¹H-NMR (DMSO-d₆):: 7,79 ppm (s, 1H); 7,71 (d, 2H); 7,61 ppm (s, 1H); 7,44 ppm (d, 2H); 3,57 ppm (s, 3H); 2,41 ppm (s, 3H).

Stufe 6: In einem 500 ml Mehrhalskolben, versehen mit Magnetrührer, Thermometer, Tropftrichter, Rückflusskühler und Stickstoffüberleitung, werden 11,1 g p-Toluolsulfonsäure-2-chlor-6-methoxy-benzthiazol-5-yl-ester, 12 g 1,3-Diethyl-2-thiobarbitursäure, 12,72 g Kaliumphosphat und 200 ml Diglyme während 18 Std auf 140°C erhitzt. Danach wird abgekühlt, auf 600 ml 6%ige wässrige Ammoniumchlorid-Lösung gegossen, die Suspension filtriert, der Rückstand mit Wasser gewaschen und 18 Std. bei 50°C /2,5 10³ Pa getrocknet. Das Rohprodukt wird mittels Flash-Chromatographie (Laufmittel Chloroform) gereinigt. Man erhält 13 g Toluol-4-sulfonsäure-2-(1,3-diethyl-4,6-dioxo-2-thioxo-tetrahydro-pyrimidin-5-yliden)-6-methoxy-2,3-dihydro-benzthiazol-5-yl-ester.
- ¹H-NMR (CDCl₃):: 13,92 ppm (s, 1H); 7,78 ppm (d, 2H); 7,41 (s, 1H); 7,35 ppm (d, 2H); 7,26 ppm (s, 1H); 7,22 ppm (s, 1H); 4,64 ppm (q, 4H); 3,69 ppm (s, 3H); 2,48 ppm (s, 3H); 1,34 (t, 6H).

Stufe 7: In einem 500 ml Mehrhalskolben, versehen mit Magnetrührer, Thermometer, Rückflusskühler und Stickstoffüberleitung, werden 12,26 g Toluol-4-sulfonsäure-2-(1,3-diethyl-4,6-dioxo-2-thioxo-tetrahydro-pyrimidin-5-yliden)-6-methoxy-2,3-dihydro-benzthiazol-5-yl-ester, 250 ml Ethanol und 6,44 g Kaliumhydroxid vorgelegt und das Raktionsgemisch während 2 Std. unter Rückfluss gekocht. Danach wird abgekühlt und auf 600 ml 6%ige wässrige Ammoniumchlorid-Lösung gegossen. Die Suspension wird filtriert, der Rückstand mit Wasser gewaschen und an der Luft getrocknet. Das Rohprodukt wird aus Ethanol umkristallisiert. Man erhält 5,1 g 1,3-Diethyl-5-(5-hydroxy-6-methoxy-3H-benzthiazol-2-yliden)-2-thioxo-dihydro-pyrimidin-4,6-dion).
- ¹H-NMR (DMSO-d₆):: 8,89 ppm (s, 1 H); 7,38 (s, 1 H); 7,23 ppm (s, 1 H); 4,48 ppm (q, 4H); 3,81 ppm (s, 3H); 1,19 (t, 6H);
- UV/VIS (Ethanol):: λₘₐₓ = 377 nm, ε = 30 080 I· mol⁻¹· cm⁻¹.

### Beispiel 2:

Stufe 1: In einem 20 l Reaktionsgefäss, versehen mit Ankerrührer, Thermometer und Stickstoffüberleitung, werden 307 g p-Anisidin in 4 l Eisessig gelöst, 760 g Natriumthiocyanat und dann 1,2 kg Kupfer(II)chlorid gelöst in 2,5 l Ethanol zugegeben. Das Gemisch wird während 70 Min. auf 75°C und dann 5 Min auf 100°C erhitzt. Anschliessend werden das Reaktionsgemisch auf 60°C abgekühlt, 8 l 1,1N HCl zugegeben und filtriert. Das Filtrat wird unter Kühlung mit 50%iger wässriger NaOH-Lösung basisch gestellt und 18 Std. bei 0°C stehen gelassen. Danach wird filtriert, portionenweise mit 8 l Wasser gewaschen und unter 5·10⁻⁶ Pa getrocknet. Man erhält 527 g rohes 2-Amino-6-methoxy-benzthiazol.
- ¹H-NMR (DMSO-d₆):: 7,3 ppm (d, 1H); 7,1 ppm (d, 1H); 6,6 ppm (s, 1H); 3,9 ppm (s, 3H).

Stufe 2: In einem 10 l Reaktionsgefäss, versehen mit Ankerrührer, Thermometer und Stickstoffüberleitung, werden 454 g rohes 2-Amino-6-methoxy-benzthiazol gemäss Stufe 1 in 1,1 l Polyethylenglykol 300 und 1,6 l Acetonitril suspendiert. Es werden 400 g Kupfer(II)chlorid, suspendiert in 1,1 l Polyethylenglykol 300 und 1,6 l Acetonitril und anschliessend 500 ml Isopentylnitrit zugegeben und das Gemisch während 3 Std. auf 65°C erwärmt. Danach wird auf 23°C abgekühlt, auf 5 I 20%ige Salzsäure gegossen und drei Mal mit je 2 l Hexan extrahiert. Die vereinigten organischen Phasen werden über Natriumsulfat getrocknet, filtriert und eingedampft. Man erhält 151 g 2-Chlor-6-methoxy-benzthiazol als leicht gelbe Kristalle.
- ¹H-NMR (CDCl₃): 7,81 ppm (d, 1H); 7,22 ppm (d, 1H); 7,08 ppm (s, 1H); 3,80 ppm (s, 3H).

Stufe 3: In einem 2,5 l Reaktionsgefäss, versehen mit Ankerrührer, Thermometer, Tropftrichter und Stickstoffüberleitung, werden 151 g 2-Chlor-6-methoxy-benzthiazol in 1,5 l Chloroform gelöst, unter Rühren auf 4°C gekühlt und innerhalb von 30 Min. 185 ml Brom zugetropft. Das Gemisch wird auf 23°C erwärmt und während 3 Std. gerührt. Danach wird unter Eiskühlung auf 3,5 l 10%ige wässrige Natriumthiosulfat-Lösung gegossen und 15 Min. gerührt. Die Phasen werden getrennt, die organische Phase mit 1 l Wasser gewaschen, über Natriumsulfat getrocknet, filtriert und eingedampft. Es werden 207 g Rohprodukt erhalten, welches mit 100 ml Hexan zum Rückfluss erhitzt und heiss filtriert wird. Man erhält 162 g 7-Brom-2-chlor-6-methoxy-benzthiazol.
- ¹H-NMR (CDCl₃): 7,82 ppm (d, 1H); 7,09 ppm (d, 1H); 3,97 ppm (s, 3H).

Stufe 4: In einem 2,5 l Reaktionsgefäss, versehen mit Ankerrührer, Thermometer und Stickstoffüberleitung, werden 33 g 7-Brom-2-chlor-6-methoxy-benzthiazol, 24,2 g 1,3-Diethyl-2-thiobarbitursäure, 25,6 g Kaliumphosphat und 1 l Diglyme während 18 Std auf 180°C erhitzt. Danach wird abgekühlt, auf 3,9 l 6%ige wässrige Ammoniumchlorid-Lösung gegossen und 30 Min. gerührt. Die flüssige Phase wird abdekantiert und der Rückstand mit 3 l Eiswasser behandelt. Der braune Feststoff wird abfiltriert und getrocknet. Man erhält 44,7 g Rohprodukt, welches mit 500 ml Hexan zum Rückfluss erhitzt und heiss filtriert wird. Nach 18 Std. Trocknung bei 50°C/2,5 10³ Pa erhält man 37,6 g 5-(7-Brom-6-methoxy-3H-benzthiazol-2-yliden)-1,3-diethyl-2-thioxo-dihydro-pyrimidin-4,6-dion als gelben Feststoff.
- ¹H-NMR (CDCl₃): 13,9 ppm (s, 1H); 7,49 ppm (d, 1H); 7,09 ppm (d, 1H); 4,66 ppm (q, 4H); 3,98 ppm (s, 3H); 1,36 ppm (t, 6H).

Stufe 5: In einem 500 ml Mehrhalskolben, versehen mit Magnetrührer, Thermometer, Tropftrichter und Stickstoffüberleitung, wird eine Suspension von 6 g Kaliumhydrid (30%ig in Mineralöl) mit 40 ml Tetrahydrofuran gewaschen. Anschliessend werden bei 0°C 13,26 g 5-(7-Brom-6-methoxy-3H-benzthiazol-2-yliden)-1,3-diethyl-2-thioxo-dihydro-pyrimidin-4,6-dion, gelöst in 200 ml Tetrahydrofuran zugegeben und 1 Std. gerührt. Anschliessend wird mit einem CO₂/Aceton-Bad auf -78°C abgekühlt und dann 40,8 ml tert.-Butyl-lithium (1,5 M in Pentan) zugegeben und 30 Min. bei dieser Temperatur gerührt. Danach werden 5,64 ml Dimethyldisulfid zugegeben und 1 Std. gerührt. Das Reaktionsgemisch wird mit 700 ml 6%iger wässriger Ammoniumchlorid-Lösung hydrolisiert und drei Mal je mit 250 ml Chloroform extrahiert. Die vereinigten organischen Phasen werden mit Wasser und gesättigter Natriumchlorid-Lösung gewaschen, über Natriumsulfat getrocknet, filtriert und eingedampft. Das Rohprodukt wird aus Ethylacetat umkristallisiert. Man erhält 9,1 g 1,3-Diethyl-5-(6-methoxy-7-methylsulfanyl-3H-benzthiazol-2-yliden)-2-thioxo-dihydro-pyrimidin-4,6-dion als gelben Feststoff.
- ¹H-NMR (CDCl₃): 7,49 ppm (d, 1H); 7,19 ppm (d, 1H); 4,66 ppm (q, 4H); 3,98 ppm (s, 3H); 2,40 ppm (s, 3H); 1,36 ppm (t, 6H)

Stufe 6: In einem 1 l Mehrhalskolben, versehen mit Magnetrührer, Thermometer, Tropftrichter und Stickstoffüberleitung, werden 9 g 1,3-Diethyl-5-(6-methoxy-7-methylsulfanyl-3H-benzthiazol-2-ylidene)-2-thioxo-dihydro-pyrimidin-4,6-dion, gelöst in 400 ml Dichlormethan vorgelegt und auf 5°C abgekühlt. Anschliessend werden 11 g Bortribromid zugetropft und anschliessend 4 Std bei 23°C gerührt. Das Reaktionsgemisch wird mit gesättigter wässriger Natriumbicarbonat-Lösung neutralisiert und die Phasen getrennt. Die wässrige Phase wird mit 400 ml Dichlormethan extrahiert, die vereinigten organischen Phasen werden mit Wasser und gesättigter wässriger Natriumchlorid-Lösung gewaschen, über Natriumsulfat getrocknet, filtriert und eingedampft. Das Rohprodukt wird mittels Flash-Chromatographie (Laufmittel Chloroform) gereinigt. Man erhält 5 g 1,3-Diethyl-5-(6-hydroxy-7-methylsulfanyl-3H-benzthiazol-2-yliden)-2-thioxo-dihydro-pyrimidin-4,6-dion als leicht gelben Feststoff.
- ¹H-NMR (CDCl₃): 7,49 ppm (d, 1H); 7,19 ppm (d, 1H); 4,66 ppm (q, 4H); 2,40 ppm (s, 3H); 1,36 ppm (t, 6H);
- UV/VIS (Ethanol):: λₘₐₓ = 385 nm, ε = 55 330 I· mol⁻¹· cm⁻¹.

### Beispiel 3:

Stufe 1: In einem 250 ml Mehrhalskolben, versehen mit Magnetrührer, Thermometer und Stickstoffüberleitung, werden 10 g 2-Methyl-benzthiazol-5-ol in 120 ml Pyridin gelöst und 60 ml Essigsäureanhydrid sowie 370 mg 4-Dimethylaminopyridin zugegeben. Die klare, farblose Lösung wird 90 Min. bei 23°C gerührt, auf 750 ml Eiswasser gegossen und die sich bildende Suspension 30 Min. gerührt. Danach wird filtriert, der Rückstand drei Mal je mit 100 ml Wasser gewaschen und 18 Std. bei 50°C /2,5 10³ Pa getrocknet. Man erhält 9,45 g Essigsäure-2-methyl-benzthiazol-5-yl-ester als weissen Feststoff.
- ¹H-NMR (CDCl₃):: 7,77 ppm (d, J = 8,8 Hz, 1H); 8,23 ppm (d, J = 2,3 Hz, 1H); 7,10 ppm (dxd, J₁ = 8,8 Hz, J₂= 2,3 Hz, 1H); 2,82 ppm (s, 3H); 2,34 ppm (s, 3H).

Stufe 2: In einem 250 ml Mehrhalskolben, versehen mit Magnetrührer, Thermometer und Stickstoffüberleitung, werden 12,52 g Triethyloxonium-tetrafluoroborat in 100 ml wasserfreiem 1,2-Dichlorethan gelöst, 11,8 g der Verbindung gemäss Stufe 1 zugegeben und die resultierende klare, farblose Lösung 18 Std. bei 23°C gerührt. Danach wird die weisse Suspension filtriert, der Rückstand zwei Mal je mit 10 ml 0°C kaltem 1,2-Dichlorethan gewaschen und 18 Std. bei 40°C/2,5· 10³ Pa getrocknet. Man erhält 16,55 g 5-Acetoxy-3-ethyl-2-methyl-benzthiazol-3-ium-tetrafluorborat als weissen Feststoff.
- ¹H-NMR (DMSO-d₆):: 8,42 ppm (d, J = 9,1 Hz, 1H); 7,65 ppm (d, J = 2,1 Hz, 1H); 7,60 ppm (dxd, J₁ = 9,1 Hz, J₂= 2,1 Hz, 1H); 4,69 ppm (q, J = 7,3 Hz, 2H); 3,19 ppm (s, 3H); 2,36 ppm (s, 3H); 1,43 ppm (t, J = 7,3 Hz, 3H).

Stufe 3: In einem 500 ml Mehrhalskolben, versehen mit Magnetrührer, Thermometer, Rückflusskühler, Septum und Stickstoffüberleitung, werden 16,5 g der Verbindung gemäss Stufe 2 in 250 ml Pyridin gelöst und die Lösung unter Rühren auf 3°C abgekühlt. Mit einer Spritze werden 7,23 g Acetylchlorid zugetropft, wobei sich ein Gel bildet. Das Reaktionsgemisch wird auf 23°C erwärmt, wobei sich wieder eine klare, braune Lösung bildet. Es wird 15 Min. bei dieser Temperatur nachgerührt, dann mit einem vorgeheizten Ölbad rasch auf 100°C Innentemperatur erhitzt und 10 Min. bei dieser Temperatur gehalten. Anschliessend wird rasch mit einem Eisbad auf etwa 25°C abgekühlt und das Pyridin bei 40°C Wasserbadtemperatur am Rotationsverdampfer eingedampft. Das Rohprodukt wird mittels Flash-Chromatographie mit Hexan/Ethylacetat (2 : 3) als Laufmittel gereinigt. Man erhält 9,86 g Essigsäure-(3-ethyl-2-[2-oxo-propyliden]-2,3-dihydro-benzthiazol-5-yl)-ester als gelben Feststoff.
- ¹H-NMR (CDCl₃):: 7,50 ppm (d, J = 8,8 Hz, 1H); 6,89 - 6,85 ppm (m, 2H); 5,87 ppm (s, 1H); 4,00 ppm (q, J = 7,3 Hz, 2H); 2,33 ppm (s, 3H); 2,24 ppm (s, 3H); 1,37 ppm (t, J = 7,3 Hz, 3H).

Stufe 4: In einem 500 ml Mehrhalskolben, versehen mit Magnetrührer, Thermometer, Tropftrichter und Stickstoffüberleitung, werden 9,8 g der Verbindung gemäss Stufe 3 in 300 ml Ethanol suspendiert und unter Rühren auf 3°C abgekühlt. Innerhalb von 20 Min. werden 38,8 ml 1N Kalilauge zugetropft, wobei die Innentemperatur bei <10°C gehalten wird. Danach wird das Eisbad entfernt und 30 Min. nachgerührt. Anschliessend werden 10 ml 4N Salzsäure zugetropft und die sich bildende Suspension auf 500 ml Phosphatpuffer pH = 6 gegossen und 15 Min. nachgerührt. Es wird filtriert, der Rückstand drei Mal je mit 100 ml Wasser gewaschen und dann 18 Std. bei 50°C /2,5·10³ Pa getrocknet. Man erhält 7,5 g 1-[3-Ethyl-5-hydroxy-3H-benzthiazol-2-yliden]-propan-2-on als weissen Feststoff.
- ¹H-NMR (DMSO-d₆):: 7,44 ppm (d, J = 8,2 Hz, 1H); 6,75 ppm (d, J = 2,4 Hz, 1H); 6,62 ppm (dxd, J₁ = 8,2 Hz, J₂ = 2,4 Hz, 1H); 6,03 ppm (s, 1 H); 4,05 ppm (q, J = 7,0 Hz, 2H); 2,07 ppm (s, 3H); 1,21 ppm (t, J = 7,0 Hz, 3H).

Stufe 5: In einem 250 ml Mehrhalskolben, versehen mit Magnetrührer, Thermometer, Rückflusskühler und Stickstoffüberleitung, werden 7,5 g der Verbindung gemäss Stufe 4 in 100 ml Bis-(dimethylamino)-methan suspendiert und unter Rühren während 26 Std. auf Rückfluss erhitzt (Innentemperatur 82°C). Anschliessend wird das Lösungsmittel abdestilliert und der Rückstand mittels Flash-Chromatographie mit Ethylacetat/Methanol (1:1) als Laufmittel gereinigt. Man erhält 6,58 g 1-[6-Dimethylaminomethyl-3-ethyl-5-hydroxy-3H-benzthiazol-2-ylidene]-propan-2-on als leicht gelben Feststoff.
- ¹H-NMR (DMSO-d₆):: 7,36 ppm (s, 1H); 6,80 ppm (s, 1H); 6,02 ppm (s, 1H); 4,06 ppm (q, J = 7,0 Hz, 2H); 3,62 ppm (s, 2H); 2,25 ppm (s, 6H); 2,07 ppm (s, 3H); 1,21 ppm (t, J = 7,0 Hz, 3H);
- UV/VIS (CH₂Cl₂):: λₘₐₓ = 365 nm, ε = 44420 I mol⁻¹ cm⁻¹.

### Beispiel 4:

Stufe 1: In einem 350 ml Sulfierkolben, versehen mit Ankerrührer, Thermometer, Tropftrichter und Stickstoffüberleitung werden 200 ml 98%ige Schwefelsäure vorgelegt und unter Rühren mit einem Kochsalz/Eisbad auf -3°C abgekühlt. Innerhalb von 25 Min. werden 20 g 6-Methoxy-2-methylbenzthiazol zugetropft, wobei die Innentemperatur unter 3°C gehalten wird. Danach wird 5 Min. nachgerührt und dann innerhalb von 40 Min. 35,16 g 20%ige Salpetersäure zugetropft, wobei die Innentemperatur unter 5°C gehalten wird. Das Reaktionsgemisch wird unter starkem Rühren auf 1 kg Eis gegossen und die sich bildende Suspension 30 Min. nachgerührt. Die Suspension wird filtriert und der Rückstand drei Mal je mit 500 ml Eiswasser gewaschen und dann 18 Std. bei 50°C/2,5·10³ Pa getrocknet. Man erhält 29,1 g eines gelben Feststoffes, welcher aus 500 ml Isopropanol umkristallisiert wird. Man erhält 17,0 g 6-Methoxy-2-methyl-7-nitro-benzthiazol als gelben Feststoff.
- ¹H-NMR (DMSO-d₆):: 8,19 ppm (d, J = 8,8 Hz, 1 H); 7,50 ppm (d, J = 8,8 Hz, 1 H); 4,04 ppm (s, 3H); 2,76 ppm (s, 3H).

Stufe 2: In einem 250 ml Mehrhalskolben, versehen mit Magnetrührer, Thermometer und Stickstoffüberleitung, werden 9,77 g Triethyloxonium-tetrafluoroborat in 90 ml wasserfreim 1,2-Dichlorethan gelöst, 10,0 g der Verbindung gemäss Stufe 1 zugegeben und die resultierende klare Lösung 18 Std. bei 23°C gerührt. Danach wird die Suspension filtriert, der Rückstand zwei Mal je mit 20 ml eiskaltem 1,2-Dichlorethan gewaschen und 18 Std. bei 45°C/2,5 10³ Pa getrocknet. Man erhält 13,19 g 3-Ethyl-6-methoxy-2-methyl-7-nitro-benzthiazol-3-ium-tetrafluorborat als weissen Feststoff.
- ¹H-NMR (DMSO-d₆):: 8,72 ppm (d, J = 9,4 Hz, 1 H); 7,97 ppm (d, J = 9,4 Hz, 1 H); 4,80 ppm (q, J = 7,3 Hz, 2H); 4,19 ppm (s, 3H); 3,21 ppm (s, 3H); 1,46 ppm (t, J = 7,3 Hz, 3H).

Stufe 3: In einem 100 ml Mehrhalskolben, versehen mit Magnetrührer, Thermometer, Rückflusskühler, Septum und Stickstoffüberleitung, werden 2,0 g der Verbindung gemäss Stufe 2 in 25 ml Pyridin gelöst und die Lösung unter Rühren auf 3°C abgekühlt. Mit einer Spritze werden 0,83 g Acetylchlorid zugetropft. Es wird 15 Min. bei dieser Temperatur nachgerührt, dann mit einem vorgeheizten Ölbad rasch auf 100°C Innentemperatur erhitzt und 10 Min. bei dieser Temperatur gehalten. Anschliessend wird rasch mit einem Eisbad auf 20°C abgekühlt, auf 200 ml gesättigte NaCl-Lösung gegossen, filtriert, gut mit Wasser gewaschen und dann 18 Std. bei 50°C/2,5 10³ Pa getrocknet. Man erhält 1,1 g eines rotbraunen Feststoffes, welcher unter Rückfluss in 15 ml Pyridin heiss gelöst wird. Die heisse Lösung wird unter Rühren auf 400 ml Wasser getropft, die sich bildende Suspension filtriert, der Rückstand drei Mal je mit 50 ml Wasser gewaschen und dann 18 Std. bei 50°C/2,5 10³ Pa getrocknet. Man erhält 0,99 g 1-[3-Ethyl-6-methoxy-7-nitro-3H-benzthiazo-yliden]-propan-2-on als rotbraunen Feststoff.
- ¹H-NMR (DMSO-d₆):: 7,73 ppm (d, J = 9,1 Hz, 1H); 7,38 ppm (d, J = 9,1 Hz, 1H); 6,10 (s, 1H); 4,16 ppm (q, J = 7,3 Hz, 2H); 3,96 ppm (s, 3H); 2,12 ppm (s, 3H); 1,22 ppm (t, J = 7,3 Hz, 3H).

Stufe 4: In einem 50 ml Mehrhalskolben, versehen mit Magnetrührer, Thermometer, Rückflusskühler und Stickstoffüberleitung werden 4,60 g wasserfreies Zinn(II)chlorid in 10 ml konz. Salzsäure gelöst, 2,1 g der Verbindung genäss Stufe 3 zugegeben und unter Rühren während 3 Std. auf 60°C Innentemperatur erwärmt. Danach wird mit einem Eisbad abgekühlt, 20 ml 10N Natronlauge zugetropft und die grünliche Suspension drei Mal je mit 100 ml Dichlormethan extrahiert. Die vereinigten organischen Phasen werden mit Wasser gewaschen bis der pH ungefähr 5 beträgt, über Magnesiumsulfat getrocknet, filtriert und eingedampft. Man erhält 1,69 g eines dunkelbraunen Feststoffes, welcher mittels Flash-Chromatographie mit Ethylacetat/ Methanol (9: 1) als Laufmittel gereinigt wird. Man erhält 1,27 g 1-[7-Amino-3-ethyl-6-methoxy-3H-benzthiazol-2-yliden]-propan-2-on als leicht gelben Feststoff.
- ¹H-NMR (DMSO-d₆):: 6,88 ppm (d, J = 8,5 Hz, 1H); 6,59 ppm (d, J = 8,5 Hz, 1H); 5,94 (s, 1 H); 5,11 ppm (s, 2H); 4,04 ppm (q, J = 7,0 Hz, 2H); 3,76 ppm (s, 3H); 2,07 ppm (s, 3H); 1,20 ppm (t, J = 7,0 Hz, 3H).

Beispiel 5: In einem 50 ml Kolben, versehen mit Magnetrührer und Stickstoffüberleitung, werden 0,5 g der Verbindung gemäss Beispiel 1 in einem Gemisch von 10 ml Ethanol und 10 ml Tetrahydrofuran vorgelegt. Zu der gelbgrünen Suspension wird eine Lösung von 164 mg Co(II)-acetat tetrahydrat in 2 ml Ethanol zugegeben, wobei sich sofort eine braune Lösung bildet, welche 18 Std. unter Stickstoff bei 23°C gerührt wird. Danach wird die beige Suspension mit einem Eisbad während 15 Min. auf 0°C abgekühlt und dann filtriert. Der Rückstand wird mit wenig eiskaltem Ethanol, zwei Mal mit Wasser gewaschen und 18 Std. bei 40°C/2,5 10³ Pa getrocknet. Man erhält 0,37 g eines beigen Feststoffes der 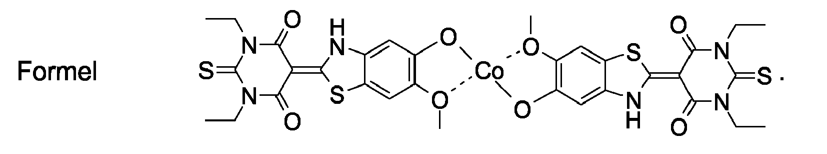
- UV/VIS (Ethanol):: λₘₐₓ = 369 nm, ε = 53 290 l·mol⁻¹·cm⁻¹.

Beispiel 6: In einem 50 ml Kolben, versehen mit Magnetrührer und Stickstoffüberleitung, werden 0,5 g der Verbindung gemäss Beispiel 2 in einem Gemisch von 10 ml Ethanol und 10 ml Tetrahydrofuran vorgelegt. Zur gelben Suspension wird eine Lösung von 157 mg Co(II)-acetat tetrahydrat in 2 ml Ethanol zugegeben. Das Reaktionsgemisch wird 18 Std. unter Stickstoff bei 23°C gerührt. Danach wird die gelbe Lösung auf 3 ml eingedampft und zu der sich bildenden Suspension 5 ml Wasser gegeben. Die Suspension wird 15 Min. gerührt und dann filtriert. Der Rückstand wird mit wenig eiskaltem Ethanol und zwei Mal mit Wasser gewaschen und 18 Std. bei 40°C/2,5·10³ Pa getrocknet. Man erhält 0,43 g eines beigen Feststoffes der Formel 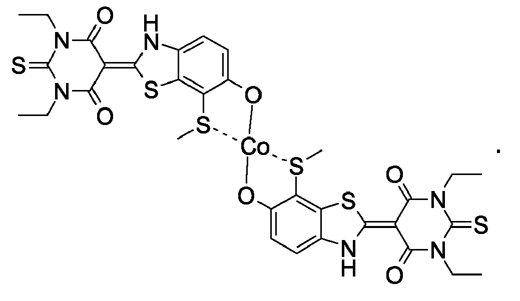
- UV/VIS (Ethanol):: λₘₐₓ = 366 nm, ε = 71 240 l·mol⁻¹·cm⁻¹.

Beispiel 7: In einem 25 ml Mehrhalskolben, versehen mit Magnetrührer, Thermometer, Septum und Stickstoffüberleitung, werden 0,5 g der Verbindung gemäss Beispiel 3 in 10 ml Ethanol vorgelegt. Zu der gelben Suspension werden mit einer Spritze 0,85 ml 2N-Natronlauge getropft, wobei sich eine gelbe Lösung bildet, zu welcher eine Lösung von 0,213 g Co(II)-acetat tetrahydrat in 2 ml Ethanol getropft wird. Die sich bildende grüne Lösung wird 18 Std. bei 23°C gerührt. Die graue Suspension wird auf etwa 3 ml eingedampft, mit 50 ml Wasser versetzt und 1 Std. gerührt. Der Rückstand wird anschliessend abfiltriert, mit Wasser gewaschen und 18 Std. bei 40°C/2,5 10³ Pa getrocknet. Man erhält 0,47 g eines grünlichen Feststoffes der Formel 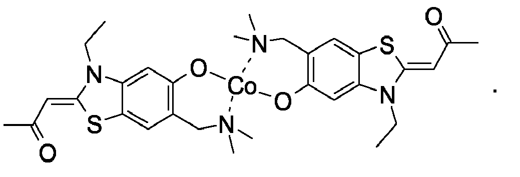
- UV/VIS (Ethanol):: λₘₐₓ = 369 nm, ε = 71 630 l·mol⁻¹·cm⁻¹.

Beispiel 8: In einem 25 ml Mehrhalskolben, versehen mit Magnetrührer, Thermometer, Septum und Stickstoffüberleitung, werden 0,5 g der Verbindung gemäss Beispiel 3 in 10 ml EtOH vorgelegt. Zur gelben Suspension werden mit einer Spritze 0,85 ml 2N Natronlauge getropft, wobei sich eine gelbe Lösung bildet, zu welcher eine Lösung von 0,212 g Ni(II)-acetat tetrahydrat in 2 ml Ethanol getropft wird. Die sich bildende olivgrüne Lösung wird 18 Std. bei 23°C gerührt, dann auf etwa 3 ml eingedampft, mit 50 ml Wasser versetzt und 1 Std. gerührt, wobei sich eine Suspension bildet. Der Rückstand wird anschliessend abfiltriert, mit Wasser gewaschen und 18 Std. bei 40°C /2,5·10³ Pa getrocknet. Man erhält 0,41 g eines hellbraunen Feststoffes der Formel 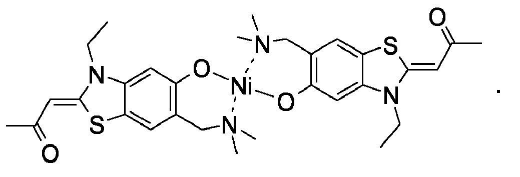
- UV/VIS (Ethanol):: λₘₐₓ = 368 nm, ε = 69 070 I·mol⁻¹·cm⁻¹.

Beispiel 9: In einem 25 ml Mehrhalskolben, versehen mit Magnetrührer, Thermometer, Septum und Stickstoffüberleitung, werden 0,5 g der Verbindung gemäss Beispiel 3 in 10 ml Ethanol vorgelegt. Zur gelben Suspension werden mit einer Spritze 0,85 ml 2N Natronlauge getropft, wobei sich eine gelbe Lösung bildet, zu welcher eine Suspension von 0,171 g Cu(II)-acetat monohydrat in 2 ml Ethanol getropft wird. Die sich bildende braune Suspension wird 18 Std bei 23°C gerührt, dann auf etwa 3 ml eingedampft, mit 50 ml Wasser versetzt und 1 Std. gerührt. Der Rückstand wird anschliessend abfiltriert, mit Wasser gewaschen und 18 Std. bei 40°C/2,5·10³ Pa getrocknet. Man erhält 0,53 g eines rotbraunen Feststoffes der Formel 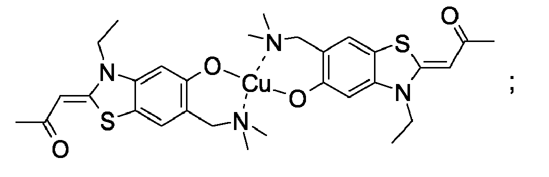
- UV/VIS (Ethanol):: λₘₐₓ = 377 nm, ε = 72 970 l· mol⁻¹· cm⁻¹.

Beispiel 10: In einem 50 ml Mehrhalskolben, versehen mit Magnetrührer, Thermometer, Septum und Stickstoffüberleitung, werden 0,422 g der Verbindung gemäss Beispiel 4 in einem Gemisch von 9 ml Ethanol und 9 ml Tetrahydrofuran vorgelegt. Zur gelben Lösung wird eine Lösung von 0,198 g Co(II)-acetat hexahydrat in 5 ml Ethanol getropft. Die klare, rotbraune Lösung wird 18 Std. bei 23°C gerührt. Das Reaktionsgemisch wird filtriert und der Rückstand mit wenig Ethanol gewaschen. Das Filtrat wird eingedampft und der Rückstand 18 Std. bei 40°C/2,5 10³ Pa getrocknet. Man erhält 0,43 g eines beigen Feststoffes der Formel 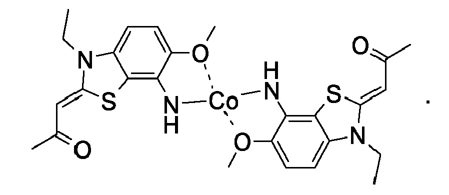
- UV/NIS (Ethanol):: λₘₐₓ = 359 nm, ε = 66400 l·mol⁻¹·cm⁻¹.

Beispiel 11: 1,0 g der Verbindung gemäss Beispiel 6 wird in 99 g 3-Methylcyclohexanon gelöst und über einen 0,45 µm Teflonfilter filtriert. Die Farbstofflösung wird anschliessend mittels Schleuderbeschichtung bei 250 U/min auf eine 1,2 mm dicke, plane Polycarbonatplatte (Durchmesser 120 mm) aufgetragen und die Drehgeschwindigkeit auf 500 U/min erhöht, so dass der Überschuss der Lösung abgeschleudert wird und eine gleichmässige Feststoffschicht entsteht. Nach dem Trocknen hat die Feststoffschicht eine Absorption von 0,16 bei 370 nm. Mittels eines optischen Messsytems (ETA-RT™, STEAG ETA-Optik) werden die Schichtdicke und der komplexe Brechungsindex ermittelt. Bei 405 nm weist die Farbstoffschicht eine Schichtdicke von 53 nm, einen Brechungsindex n von 1,92 und einen Extinktionskoeffizient k von 0,097 auf.

Beispiel 12: Analog zu Beispiel 11 wird 1 g der Verbindung gemäss Beispiel 9 in 99 g 2,2,3,3-Tetrafluor-1-propanol gelöst und über einen 0,45 µm Teflonfilter filtriert. Die Farbstofflösung wird anschliessend mittels Schleuderbeschichtung bei 250 U/min auf eine 1,2 mm dick plane Polycarbonatplatte (Durchmesser 120 mm) aufgetragen und die Drehgeschwindigkeit auf 700 U/min erhöht, so dass der Überschuss der Lösung abgeschleudert wird und eine gleichmässige Feststoffschicht entsteht. Nach dem Trocknen hat die Feststoffschicht eine Absorption von 0,45 bei 377 nm. Mittels eines optischen Messsystems (ETA-RT™, STEAG ETA-Optik) werden die Schichtdicke und der komplexe Brechungsindex ermittelt. Bei 405 nm weist die Schicht eine Schichtdicke von 40 nm, ein Brechungsindex n von 2,10 und ein Extinktionskoeffizient k von 0,135 auf.

Mit den erfindungsgemässen Medien erreicht man gleichzeitig hohe Modulation, hohe Reflexion und hohe Sensitivität bei tieferen Laserenergie.

## Patentansprüche

1. Optisches Aufzeichnungsmedium, enthaltend ein Substrat, eine Aufzeichnungsschicht und gegebenenfalls eine reflektierende Schicht, **dadurch gekennzeichnet, dass** die Aufzeichnungsschicht eine Verbindung der Formel M^{m+}(Xⁿ)ₓ(L₁)(L₂)_{y}(L₃)_{z} (I) enthält, worin L₁ und L₂ unabhängig voneinander je für einen Liganden der Formel 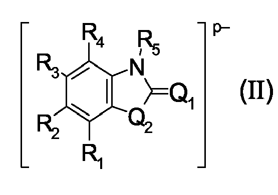 stehen, wobei
L₁ und L₂ über eine Direktbindung oder eine Verbrückung mit C₂-C₁₀Alkylen oder C₂-C₁₀Alkenylen zwischen zwei Radikalen L₁ und L₂ miteinander gebunden sein können;
L₃ unabhängig von L₁ und L₂ für einen weiteren Liganden der Formel (II) steht;
M ein Übergangsmetall der Gruppen 6 bis 12 oder ein Element der Gruppe 13 ist, welches an mindestens einem von R₁, R₂, R₃ und R₄ gebunden ist und zusätzlich mit einem oder mehreren weiteren Liganden koordiniert sein kann und/oder gegebenenfalls zum Ausgleich einer überschüssigen Ladung mit einem oder mehreren weiteren Ionen innerhalb oder ausserhalb der Koordinationssphäre eine elektrostatische Wechselwirkung haben kann;
m eine Zahl 1, 2 oder 3 ist; n eine Ladung -2, -1, +1 oder +2 ist; p eine Zahl 1 oder 2 ist; q, x, y und z unabhängig voneinander eine Zahl 0 oder 1 bedeuten, mit der Bedingung, dass die Summe der Ladungen von M^{m+}, (Xⁿ)ₓ, (L₁), (L₂)_{y} und (L₃)_{z} gleich 0 ist;
Q₁ für CR₆R₇, Q₂ für CR₈R₉, N-OR₈, N-NR₈R₉, O, S oder NR₈, und Q₃ für CR₁₀R₁₁, C=O, S=O, O=S=O oder P(O)R₁₂ stehen;
entweder eins von R₁, R₂, R₃ und R₄, oder zwei von R₁, R₂, R₃ und R₄ in einem Paar R₁ und R₂, R₂ und R₃ oder R₃ und R₄ je unabhängig voneinander (Q₃)_{q}R₁₃ sind, und die weiteren zwei oder drei von R₁, R₂, R₃ und R₄ unabhängig voneinander R₁₄, NR₁₀NR₈R₉, NO₂, SiR₈R₁₅R₁₆, C(R₁₀)=NR₈, C(R₁₀)=N-OR₈, CON(R₁₀)OR₈, CON(R₁₀)NR₈R₉, S(O)R₁₅, S(O)₂-R₁₅, S(O)-OR₈, S(O)N(R₁₀)NR₈R₉, SO₂NR₈R₉, SO₂N(R₁₀)NR₈R₉, SO₃R₈, P(O)R₁₅R₁₆, P(O)R₁₅OR₈, P(O)OR₈OR₉ oder P(O)(NR₈R₉)₂ bedeuten, wobei eins der weiteren zwei oder drei R₁, R₂, R₃ und R₄ darüber hinaus unsubstituiertes oder mit einem oder mehreren, gegebenenfalls identischen oder unterschiedlichen Resten Halogen, OH, OR₁₅, SR₁₅, NH₂, NHR₁₅, NR₁₅R₁₆, Nitro, CN, OCN, SCN, CHO, COR₁₅, CR₁₇OR₁₅OR₁₆, COOH, COOR₁₅, CONH₂, CONHR₁₅, CONR₁₅R₁₆, SO₂R₁₅, P(O)R₁₅R₁₆, P(O)R₁₅OR₁₆ und/oder P(O)OR₁₅OR₁₆ substituiertes C₆-C₁₀Aryl, C₁-C₉Heteroaryl, C₇-C₁₂Aralkyl oder C₂-C₁₂Heteroaralkyl sein kann;
oder zwei der weiteren zwei oder drei R₁, R₂, R₃ und R₄ stehen als Paar R₁ und R₂, R₂ und R₃ oder R₃ und R₄ zu zweit zusammen für unsubstituiertes oder mit NR₁₅R₁₆, SR₁₅, Halogen und/oder OR₁₅ ein- oder mehrfach substituiertes C₂-C₁₀Alkylen oder C₂-C₁₀Alkenylen, wobei ein zusätzlicher Ring gebildet wird, welcher bevorzugt nicht durchkonjugiert ist;
R₅, R₈, R₉, R₁₀ und R₁₁ unabhängig voneinander und unabhängig von R₁ bis R₄, R₆, R₇ und R₁₂ bis R₁₉ für Wasserstoff, für unsubstituiertes oder mit NR₁₅R₁₆, SR₁₅, Halogen und/oder OR₁₅ ein- oder mehrfach substituiertes C₁-C₅Alkyl, C₂-C₅Alkenyl, C₂-C₅Alkinyl, C₃-C₅Cycloalkyl, Hetero-C₂-C₅cycloalkyl oder C₃-C₅Cycloalkenyl, oder für unsubstituiertes oder mit einem oder mehreren, gegebenenfalls identischen oder unterschiedlichen Resten Halogen, OH, OR₁₅, SR₁₅, NH₂, NHR₁₅, NR₁₅R₁₆, Nitro, CN, OCN, SCN, CHO, COR₁₅, CR₁₇OR₁₅OR₁₆, COOH, COOR₁₅, CONH₂, CONHR₁₅, CONR₁₅R₁₆, SO₂R₁₅, P(O)R₁₅R₁₆, P(O)R₁₅OR₁₆ und/oder P(O)OR₁₅OR₁₆ substituiertes C₆-C₁₀Aryl, C₁-C₉Heteroaryl, C₇-C₁₂Aralkyl oder C₂-C₁₂Heteroaralkyl stehen; oder R₈ und R₉ und/oder R₁₀ und R₁₁ stehen zusammen für unsubstituiertes oder mit NR₁₅R₁₆, SR₁₅, Halogen und/oder OR₁₅ ein- oder mehrfach substituiertes C₂-C₁₀Alkylen oder C₂-C₁₀Alkenylen, welches durch O oder NR₁₅ unterbrochen sein kann;
R₆ für Wasserstoff, R₁₈, OR₈, SR₈, NR₈R₉; für unsubstituiertes oder mit COR₁₀, COOR₁₀, CONR₈R₉, CN, Halogen und/oder OR₁₅ ein- oder mehrfach substituiertes C₁-C₅Alkyl, C₂-C₅Alkenyl, C₂-C₅Alkinyl, C₃-C₅Cycloalkyl, Hetero-C₂-C₅cycloalkyl oder C₃-C₅Cycloalkenyl steht;
R₇ und R₁₈ unabhängig voneinander und unabhängig von R₁ bis R₆, R₈ bis R₁₇ und R₁₉ für CN, COR₁₀, C(R₁₀)=NR₂₀, COOR₁₀, CONR₈R₉, SOR₁₂, SO₂R₁₂ oder SO₂NR₈R₉ stehen;
wobei R₆ und R₇ gegebenenfalls über eine Direktbindung, O, S, NR₈, C=O, C=S oder C=NR₈ zusammen gebunden sein können, so dass sich für Q₁ ein 5-, 6- oder 7-gliedriger Ring ergibt;
R₁₂ für OR₈, SR₈, NR₈R₉ oder R₁₁ steht;
R₁₃ für O⁻, S⁻ oder N⁻R₂₁ steht;
R₁₄, gegebenenfalls jedes R₁₄ unabhängig von anderen R₁₄, für Wasserstoff, Halogen, OR₁₉, SR₁₉, NR₈R₉, COR₁₀, COOR₁₀, CONR₈R₉, CN oder für unsubstituiertes oder mit NR₁₅R₁₆, SR₁₅, Halogen und/oder OR₁₅ ein- oder mehrfach substituiertes C₁-C₅Alkyl, C₂-C₅Alkenyl, C₂-C₅Alkinyl, C₃-C₅Cycloalkyl, Hetero-C₂-C₅cycloalkyl oder C₃-C₅Cycloalkenyl steht;
R₁₅, R₁₆ und R₁₇ unabhängig voneinander für unsubstituiertes oder mit NR₂₂R₂₃, SR₂₂, Halogen und/oder OR₂₂ ein- oder mehrfach substituiertes C₁-C₅Alkyl, C₂-C₅Alkenyl, C₂-C₅Alkinyl, C₃-C₅Cycloalkyl, Hetero-C₂-C₅cycloalkyl oder C₃-C₅Cycloalkenyl stehen, oder R₁₅ und R₁₆ stehen zusammen für unsubstituiertes oder mit NR₂₂R₂₃, SR₂₂, Halogen und/oder OR₂₂ ein- oder mehrfach substituiertes C₂-C₁₀Alkylen oder C₂-C₁₀Alkenylen, welches durch O oder NR₂₂ unterbrochen sein kann;
R₁₉ für Wasserstoff, COR₁₀, COOR₂₄, CR₈OR₉OR₁₀, CN, CONR₈R₉, SO₂R₂₄, P(O)R₂₄R₂₅, P(O)R₂₄OR₂₅, P(O)OR₂₄OR₂₅ oder für unsubstituiertes oder mit NR₁₅R₁₆, SR₁₅, Halogen und/oder OR₁₅ ein- oder mehrfach substituiertes C₁-C₅Alkyl, C₂-C₅Alkenyl, C₂-C₅Alkinyl, C₃-C₅Cycloalkyl, Hetero-C₂-C₅cycloalkyl oder C₃-C₅Cycloalkenyl steht;
R₂₀ für NR₈R₉, OR₉, SR₉, R₈ oder R₂₁ steht;
R₂₁ für COR₁₀, COOR₂₄, CN, CONR₈R₉, SO₂R₂₄, P(O)R₂₄R₂₅, P(O)R₂₄OR₂₅ oder P(O)OR₂₄OR₂₅ steht;
R₂₂ und R₂₃ unabhängig voneinander und unabhängig von R₁ bis R₁₉ für Wasserstoff, für unsubstituiertes oder mit Halogen, OR₂₆ und/oder NR₂₆R₂₇ ein- oder mehrfach substituiertes C₁-C₅Alkyl, C₂-C₅Alkenyl , C₂-C₅Alkinyl, C₃-C₅Cycloalkyl, Hetero-C₂-C₅cycloalkyl oder C₃-C₅Cycloalkenyl, oder für unsubstituiertes oder mit einem oder mehreren, gegebenenfalls identischen oder unterschiedlichen Resten Halogen, OR₂₆, NR₂₆R₂₇, Nitro, CN, OCN, SCN, COR₂₆, CR₂₆OR₂₈OR₂₉, COOR₂₈, CONR₂₆R₂₇, SO₂R₂₈, P(O)R₂₈R₂₉, P(O)R₂₈OR₂₉ und/oder P(O)OR₂₈OR₂₉ substituiertes Phenyl, C₁-C₄Heteroaryl, Benzyl oder C₂-C₅Heteroaralkyl stehen; oder R₂₂ und R₂₃ stehen zusammen für unsubstituiertes oder mit NR₂₆R₂₇, SR₂₆, Halogen und/oder OR₂₆ ein- oder mehrfach substituiertes C₂-C₅Alkylen oder C₂-C₅Alkenylen, welches durch O oder NR₂₆ unterbrochen sein kann;
R₂₄ und R₂₅ unabhängig voneinander für unsubstituiertes oder mit NR₂₆R₂₇, SR₂₆, Halogen und/oder OR₂₆ ein- oder mehrfach substituiertes C₁-C₅Alkyl, C₂-C₅Alkenyl, C₂-C₅Alkinyl, C₃-CₛCycloalkyl, Hetero-C₂-Cₛcycloalkyl oder C₃-C₅Cycloalkenyl stehen, oder R₂₄ und R₂₅ stehen zusammen für unsubstituiertes oder mit NR₂₆R₂₇, SR₂₆, Halogen und/oder OR₂₆ ein- oder mehrfach substituiertes C₂-C₁₀Alkylen oder C₂-C₁₀Alkenylen, welches durch O oder NR₂₆ unterbrochen sein kann;
R₂₆ und R₂₇ unabhängig voneinander für Wasserstoff, Methyl oder Ethyl, sowie R₂₈ und R₂₉ unabhängig voneinander für Methyl oder Ethyl stehen; oder R₂₆ und R₂₇ und/oder R₂₈ und R₂₉ stehen zusammen für unsubstituiertes oder durch Methyl und/oder Ethyl 1- bis 5-fach substituiertes 1,5-Pentylen, 3-Oxa-1,5-pentylen oder 3-Oxa-1,5-pentylen; und
Xⁿ ein Gegenion bedeutet.

2. Optisches Aufzeichnungsmedium gemäss Anspruch 1, worin M für Au, Cd, Co, Cu, Cr, Ir, Mn, Mo, Ni, Fe, Os, Pd, Pt, Re, Rh, Ru, W oder Zn, bevorzugt für Co, Cu oder Ni, besonders bevorzugt für Co(II), Cu(II) oder Ni(II) steht.

3. Optisches Aufzeichnungsmedium gemäss Anspruch 1 oder 2, worin n die Zahl 2 ist und Q₂ für CR₈R₉, S, O oder NR₁₉, bevorzugt für S steht.

4. Optisches Aufzeichnungsmedium gemäss Anspruch 1, 2 oder 3, worin das Übergangsmetall M mit (Q₃)_{q}R₁₃und einem Heteroatom N, O oder S auf einem vicinalen Rest R₁, R₂, R₃ oder R₄ einen 5-, 6- oder 7-gliedriger Komplex bildet.

5. Optisches Aufzeichnungsmedium gemäss Anspruch 1, 2, 3 oder 4, worin die Verbindung der Formel (I) eine Teilstruktur M^{m+}(L₁) der Formel 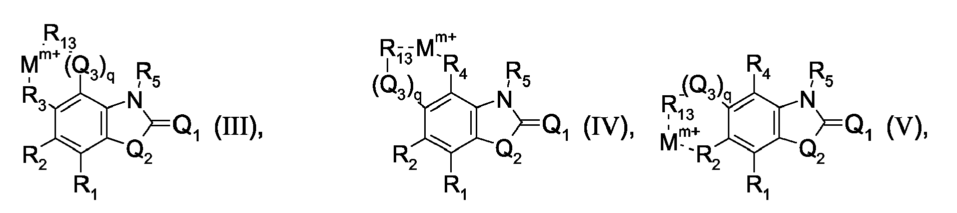 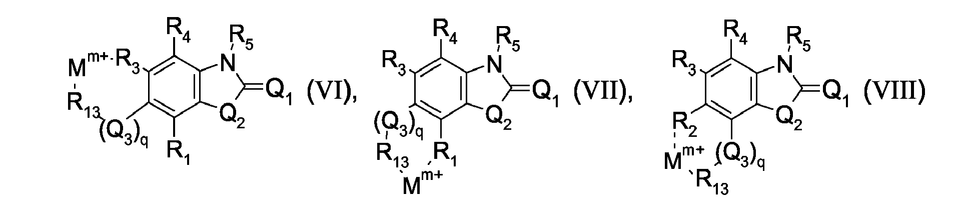 oder 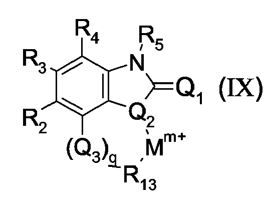 aufweist.

6. Optisches Aufzeichnungsmedium gemäss Anspruch 1, 2, 3, 4 oder 5, worin R₁₀ unsubstituiertes oder mit Halogen und/oder OR₁₅ ein- oder mehrfach substituiertes C₁-C₅Alkyl, C₂-C₅Alkenyl, C₂-C₅Alkinyl, C₃-C₅Cycloalkyl, Hetero-C₂-C₅cycloalkyl oder C₃-C₅Cycloalkenyl ist.

7. Optisches Aufzeichnungsmedium gemäss Anspruch 1, 2, 3, 4, 5 oder 6, worin Alkyl, Alkenyl, Alkinyl, Cycloalkyl, Heterocycloalkyl oder Cycloalkenyl für Methyl, Ethyl, n-Propyl, Isopropyl, Vinyl, Allyl, Propargyl, Cyclopropyl, 2-Oxacydopropyl oder 2-Thiacyclopropyl steht.

8. Verfahren zur Aufzeichnung oder Wiedergabe von Daten, **dadurch gekennzeichnet, dass** die Daten auf einem optischen Aufzeichnungsmedium gemäss Anspruch 1, 2, 3, 4, 5, 6 oder 7 bei einer Wellenlänge von 300 bis 500 nm aufgezeichnet oder wiedergegeben werden.

9. Verbindung der Formel (I) gemäss Anspruch 1, 2, 3, 4, 5, 6 oder 7.

10. Verwendung einer Verbindung gemäss Anspruch 9 oder 3 zur technischen Verbesserung von optischen Aufzeichnungsmitteln.
